# EUROPEAN PATENT APPLICATION

(11) **EP 2 431 458 A1**
(43) Date of publication of application: **21.03.2012**
(21) Application number: 11008304.5
(22) Date of filing: 27.06.2006
(51) Int. Cl.: C12N 5/00, C12N 5/02, C12N 15/82, C07K 14/79

(54) **Components of cell culture media produced from plant cells**

(30) Priority: 28.06.2005 US 694236 P
(62) Divisional of application: 10161455.0
(71) Applicant: Ventria Bioscience, Sacramento, CA 95834 (US)
(72) Inventor: Deeter, Scott, Fort Collins Colorado 80525 (US); Schmidt, Joseph E., El Macero California 95618 (US); Huang, Ning, Davis California 95618 (US); Mabery, Kenneth J., Antelope California 95843 (US); Bethell, Delia R., West Sacramento California 95605 (US)
(74) Representative: Müller-Boré & Partner Patentanwälte

(57) **Abstract**

The present invention comprises the production of components of cell culture media produced from plant cells. Heterologous DNA comprising genes encoding the desired component are introduced into plant cells, especially rice, which then produce the desired component. The component can be isolated from the plant cell and combined with other components to form the required cell culture medium.

SEQUENCE LISTING

<110>  VENTRIA BIOSCIENCE

<120>  Components of Cell Culture Media Produced from Plant Cells

<130>  V 1566EU

<140>  EP10161455.0
<141>  2006-05-27

<150>  US60/694,236
<151>  2005-06-28

<160>  19

<170>  PatentIn version 3.3

<210>  1
<211>  162
<212>  DNA
<213>  Artificial

<220>
<223>  Synthetic codon optimized (egfactor) sequence


<220>
<221>  CDS
<222>  (1)..(159)

<400>  1
aac tcc gac tcg gag tgc ccc ctc tcc cac gac ggt tac tgc ctc cac        48
Asn Ser Asp Ser Glu Cys Pro Leu Ser His Asp Gly Tyr Cys Leu His
1               5                   10                  15

gac ggg gtc tgc atg tac atc gag gcc ctc gac aag tac gcc tgc aac        96
Asp Gly Val Cys Met Tyr Ile Glu Ala Leu Asp Lys Tyr Ala Cys Asn
            20                  25                  30

tgc gtc gtg ggc tac atc ggc gag cgg tgc cag tac cgc gac ctc aag        144
Cys Val Val Gly Tyr Ile Gly Glu Arg Cys Gln Tyr Arg Asp Leu Lys
        35                  40                  45

tgg tgg gag ctg cgc tga                                                162
Trp Trp Glu Leu Arg
    50


<210>  2
<211>  53
<212>  PRT
<213>  Artificial

<220>
<223>  Synthetic Construct

<400>  2

Asn Ser Asp Ser Glu Cys Pro Leu Ser His Asp Gly Tyr Cys Leu His
1               5                   10                  15


Asp Gly Val Cys Met Tyr Ile Glu Ala Leu Asp Lys Tyr Ala Cys Asn
            20                  25                  30


Cys Val Val Gly Tyr Ile Gly Glu Arg Cys Gln Tyr Arg Asp Leu Lys

35                  40                  45

Trp Trp Glu Leu Arg
    50

<210>  3
<211>  53
<212>  PRT
<213>  Artificial

<220>
<223>  Synthetic codon optimized (egfactor) sequence

<400>  3

Asn Ser Asp Ser Glu Cys Pro Leu Ser His Asp Gly Tyr Cys Leu His
1               5                   10                  15

Asp Gly Val Cys Met Tyr Ile Glu Ala Leu Asp Lys Tyr Ala Cys Asn
            20                  25                  30

Cys Val Val Gly Tyr Ile Gly Glu Arg Cys Gln Tyr Arg Asp Leu Lys
        35                  40                  45

Trp Trp Glu Leu Arg
    50

<210>  4
<211>  213
<212>  DNA
<213>  Artificial

<220>
<223>  Synthetic codon optimized (Insgfact) sequence

<220>
<221>  CDS
<222>  (1)..(210)

<400>  4
ggc cca gag acc ctg tgc ggt gcg gag ctg gtg gac gcc ctc cag ttc      48
Gly Pro Glu Thr Leu Cys Gly Ala Glu Leu Val Asp Ala Leu Gln Phe
1               5                   10                  15

gtc tgc ggg gac cgg ggc ttc tac ttc aac aag cca acg ggc tac ggg      96
Val Cys Gly Asp Arg Gly Phe Tyr Phe Asn Lys Pro Thr Gly Tyr Gly
            20                  25                  30

tcc tcc tcg cgc cgc gcc ccc cag acc ggc atc gtg gac gag tgc tgc      144
Ser Ser Ser Arg Arg Ala Pro Gln Thr Gly Ile Val Asp Glu Cys Cys
        35                  40                  45

ttc cgc tcc tgc gac ctc cgg cgg ctg gag atg tac tgc gcc cca ctc      192
Phe Arg Ser Cys Asp Leu Arg Arg Leu Glu Met Tyr Cys Ala Pro Leu
    50                  55                  60

aag ccc gcc aag agc gcc tga                                          213
Lys Pro Ala Lys Ser Ala
65                  70

<210>  5
<211>  70
<212>  PRT
<213>  Artificial

<220>
<223>  Synthetic Construct

<400>  5

Gly Pro Glu Thr Leu Cys Gly Ala Glu Leu Val Asp Ala Leu Gln Phe
1               5                   10                  15

Val Cys Gly Asp Arg Gly Phe Tyr Phe Asn Lys Pro Thr Gly Tyr Gly
            20                  25                  30

Ser Ser Ser Arg Arg Ala Pro Gln Thr Gly Ile Val Asp Glu Cys Cys
        35                  40                  45

Phe Arg Ser Cys Asp Leu Arg Arg Leu Glu Met Tyr Cys Ala Pro Leu
    50                  55                  60

Lys Pro Ala Lys Ser Ala
65                  70

<210>  6
<211>  70
<212>  PRT
<213>  Artificial

<220>
<223>  Synthetic codon optimized (Insgfact) sequence

<400>  6

Gly Pro Glu Thr Leu Cys Gly Ala Glu Leu Val Asp Ala Leu Gln Phe
1               5                   10                  15

Val Cys Gly Asp Arg Gly Phe Tyr Phe Asn Lys Pro Thr Gly Tyr Gly
            20                  25                  30

Ser Ser Ser Arg Arg Ala Pro Gln Thr Gly Ile Val Asp Glu Cys Cys
        35                  40                  45

Phe Arg Ser Cys Asp Leu Arg Arg Leu Glu Met Tyr Cys Ala Pro Leu
    50                  55                  60

Lys Pro Ala Lys Ser Ala
65                  70

<210>  7
<211>  183
<212>  DNA
<213>  Artificial

<220>
<223>  Synthetic codon optimized (Trefoil) sequence

<220>
<221>  CDS
<222>  (1)..(180)

<400>  7
gag gag tac gtc ggg ctc tcc gct aac caa tgc gcg gtc ccg gcc aag      48
Glu Glu Tyr Val Gly Leu Ser Ala Asn Gln Cys Ala Val Pro Ala Lys
1               5                   10                  15

gac cgg gtg gac tgc ggc tac ccc cac gtg acg ccg aag gag tgc aac      96
Asp Arg Val Asp Cys Gly Tyr Pro His Val Thr Pro Lys Glu Cys Asn
            20                  25                  30

aac cgg ggc tgc tgc ttc gac tcc cgc atc cca ggc gtg ccg tgg tgc      144
Asn Arg Gly Cys Cys Phe Asp Ser Arg Ile Pro Gly Val Pro Trp Cys
        35                  40                  45

ttc aag ccc ctc acc cgc aag acg gag tgc acg ttc tga                  183
Phe Lys Pro Leu Thr Arg Lys Thr Glu Cys Thr Phe
    50                  55                  60


<210>  8
<211>  60
<212>  PRT
<213>  Artificial

<220>
<223>  Synthetic Construct

<400>  8

Glu Glu Tyr Val Gly Leu Ser Ala Asn Gln Cys Ala Val Pro Ala Lys
1               5                   10                  15


Asp Arg Val Asp Cys Gly Tyr Pro His Val Thr Pro Lys Glu Cys Asn
            20                  25                  30


Asn Arg Gly Cys Cys Phe Asp Ser Arg Ile Pro Gly Val Pro Trp Cys
        35                  40                  45


Phe Lys Pro Leu Thr Arg Lys Thr Glu Cys Thr Phe
    50                  55                  60


<210>  9
<211>  60
<212>  PRT
<213>  Artificial

<220>
<223>  Synthetic codon optimized (Trefoil) sequence

<400>  9

Glu Glu Tyr Val Gly Leu Ser Ala Asn Gln Cys Ala Val Pro Ala Lys
1               5                   10                  15


Asp Arg Val Asp Cys Gly Tyr Pro His Val Thr Pro Lys Glu Cys Asn
            20                  25                  30


Asn Arg Gly Cys Cys Phe Asp Ser Arg Ile Pro Gly Val Pro Trp Cys

35                  40                  45

Phe Lys Pro Leu Thr Arg Lys Thr Glu Cys Thr Phe
    50                  55                  60

<210>  10
<211>  159
<212>  DNA
<213>  Homo sapiens

<400>  10
aatggtgact ctgaatgtcc cctgtcccac gatgggtact gcctccatga tggtgtgtgc      60

atgtatattg aagcattgga caagtatgca tgcaactgtg ttgttggcta catcggggag     120

cgatctcagt accgagacct gaagtggtgg gaactgcgc                            159

<210>  11
<211>  209
<212>  DNA
<213>  Homo sapiens

<400>  11
ggaccggaga cgctctgcgg ggctgagctg gtggatgctc ttcagttcgt gtgtggagac      60

aggggctttt atttcaacaa gcccacaggg tatggctcca gcagtcggag ggcgcctcag     120

acaggcatcg tggatgagtg ctgcttccgg agctgtgatc taaggaggct ggagatgtat     180

tgcgcacccc tcaagcctgc caagtcagc                                       209

<210>  12
<211>  183
<212>  DNA
<213>  Homo sapiens

<400>  12
gaggagtacg tgggcctgtc tgcaaaccag tgtgccgtgc cggccaagga cagggtggac      60

tgcggctacc cccatgtcac ccccaaggag tgcaacaacc ggggctgctg ctttgactcc     120

aggatccctg gagtgccttg gtgtttcaag cccctgacta ggaagacaga atgcaccttc     180

tga                                                                   183

<210>  13
<211>  2079
<212>  DNA
<213>  Homo sapiens

<220>
<221>  CDS
<222>  (1)..(2076)

<400>  13
ggc cgt agg aga agg agt gtt cag tgg tgc gcc gta tcc caa ccc gag       48
Gly Arg Arg Arg Arg Ser Val Gln Trp Cys Ala Val Ser Gln Pro Glu
1               5                   10                  15

gcc aca aaa tgc ttc caa tgg caa agg aat atg aga aaa gtg cgt ggc       96
Ala Thr Lys Cys Phe Gln Trp Gln Arg Asn Met Arg Lys Val Arg Gly
            20                  25                  30

cct cct gtc agc tgc ata aag aga gac tcc ccc atc cag tgt atc cag 144
Pro Pro Val Ser Cys Ile Lys Arg Asp Ser Pro Ile Gln Cys Ile Gln
        35                  40                  45

gcc att gcg gaa aac agg gcc gat gct gtg acc ctt gat ggt ggt ttc 192
Ala Ile Ala Glu Asn Arg Ala Asp Ala Val Thr Leu Asp Gly Gly Phe
    50                  55                  60

ata tac gag gca ggc ctg gcc ccc tac aaa ctg cga cct gta gcg gcg 240
Ile Tyr Glu Ala Gly Leu Ala Pro Tyr Lys Leu Arg Pro Val Ala Ala
65                  70                  75                  80

gaa gtc tac ggg acc gaa aga cag cca cga act cac tat tat gcc gtg 288
Glu Val Tyr Gly Thr Glu Arg Gln Pro Arg Thr His Tyr Tyr Ala Val
                85                  90                  95

gct gtg gtg aac aag ggc ggc agc ttt cag ctg aac gaa ctg caa ggt 336
Ala Val Val Asn Lys Gly Gly Ser Phe Gln Leu Asn Glu Leu Gln Gly
            100                 105                 110

ctg aag tcc tgc cac aca ggc ctt cgc agg acc gct gga tgg aat gtc 384
Leu Lys Ser Cys His Thr Gly Leu Arg Arg Thr Ala Gly Trp Asn Val
        115                 120                 125

cct ata ggg aca ctt cgt cca ttc ttg aat tgg acg ggt cca cct gag 432
Pro Ile Gly Thr Leu Arg Pro Phe Leu Asn Trp Thr Gly Pro Pro Glu
    130                 135                 140

ccc att gag gca gct gtg gcc agg ttc ttc tca gcc agc tgt gtt ccc 480
Pro Ile Glu Ala Ala Val Ala Arg Phe Phe Ser Ala Ser Cys Val Pro
145                 150                 155                 160

ggt gca gat aaa gga cag ttc ccc aac ctg tgt cgc ctg tgt gcg ggg 528
Gly Ala Asp Lys Gly Gln Phe Pro Asn Leu Cys Arg Leu Cys Ala Gly
                165                 170                 175

aca ggg gaa aac aaa tgt gcc ttc tcc tcc cag gaa ccg tac ttc agc 576
Thr Gly Glu Asn Lys Cys Ala Phe Ser Ser Gln Glu Pro Tyr Phe Ser
            180                 185                 190

tac tct ggt gcc ttc aag tgt ctg aga gac ggg gct gga gac gtg gct 624
Tyr Ser Gly Ala Phe Lys Cys Leu Arg Asp Gly Ala Gly Asp Val Ala
        195                 200                 205

ttt atc aga gag agc aca gtg ttt gag gac ctg tca gac gag gct gaa 672
Phe Ile Arg Glu Ser Thr Val Phe Glu Asp Leu Ser Asp Glu Ala Glu
    210                 215                 220

agg gac gag tat gag tta ctc tgc cca gac aac act cgg aag cca gtg 720
Arg Asp Glu Tyr Glu Leu Leu Cys Pro Asp Asn Thr Arg Lys Pro Val
225                 230                 235                 240

gac aag ttc aaa gac tgc cat ctg gcc cgg gtc cct tct cat gcc gtt 768
Asp Lys Phe Lys Asp Cys His Leu Ala Arg Val Pro Ser His Ala Val
                245                 250                 255

gtg gca cga agt gtg aat ggc aag gag gat gcc atc tgg aat ctt ctc 816
Val Ala Arg Ser Val Asn Gly Lys Glu Asp Ala Ile Trp Asn Leu Leu
            260                 265                 270

cgc cag gca cag gaa aag ttt gga aag gac aag tca ccg aaa ttc cag 864
Arg Gln Ala Gln Glu Lys Phe Gly Lys Asp Lys Ser Pro Lys Phe Gln
        275                 280                 285

ctc ttt ggc tcc cct agt ggg cag aaa gat ctg ctg ttc aag gac tct 912
Leu Phe Gly Ser Pro Ser Gly Gln Lys Asp Leu Leu Phe Lys Asp Ser
    290                 295                 300

gcc att ggg ttt tcg agg gtg ccc ccg agg ata gat tct ggg ctg tac 960
Ala Ile Gly Phe Ser Arg Val Pro Pro Arg Ile Asp Ser Gly Leu Tyr
305                 310                 315                 320

ctt ggc tcc ggc tac ttc act gcc atc cag aac ttg agg aaa agt gag 1008
Leu Gly Ser Gly Tyr Phe Thr Ala Ile Gln Asn Leu Arg Lys Ser Glu
                325                 330                 335

gag gaa gtg gct gcc cgg cgt gcg cgg gtc gtg tgg tgt gcg gtg ggc 1056
Glu Glu Val Ala Ala Arg Arg Ala Arg Val Val Trp Cys Ala Val Gly
            340                 345                 350

gag cag gag ctg cgc aag tgt aac cag tgg agt ggc ttg agc gaa ggc 1104
Glu Gln Glu Leu Arg Lys Cys Asn Gln Trp Ser Gly Leu Ser Glu Gly
        355                 360                 365

agc gtg acc tgc tcc tcg gcc tcc acc aca gag gac tgc atc gcc ctg 1152
Ser Val Thr Cys Ser Ser Ala Ser Thr Thr Glu Asp Cys Ile Ala Leu
    370                 375                 380

gtg ctg aaa gga gaa gct gat gcc atg agt ttg gat gaa gga tat gtg 1200
Val Leu Lys Gly Glu Ala Asp Ala Met Ser Leu Asp Glu Gly Tyr Val
385                 390                 395                 400

tac act gca ggc aaa tgt ggt ttg gtg cct gtc ctg gca gag aac tac 1248
Tyr Thr Ala Gly Lys Cys Gly Leu Val Pro Val Leu Ala Glu Asn Tyr
                405                 410                 415

aaa tcc caa caa agc agt gac cct gat cct aac tgt gtg gat aga cct 1296
Lys Ser Gln Gln Ser Ser Asp Pro Asp Pro Asn Cys Val Asp Arg Pro
            420                 425                 430

gtg gaa gga tat ctt gct gtg gcg gtg gtt agg aga tca gac act agc 1344
Val Glu Gly Tyr Leu Ala Val Ala Val Val Arg Arg Ser Asp Thr Ser
        435                 440                 445

ctt acc tgg aac tct gtg aaa ggc aag aag tcc tgc cac acc gcc gtg 1392
Leu Thr Trp Asn Ser Val Lys Gly Lys Lys Ser Cys His Thr Ala Val
    450                 455                 460

gac agg act gca ggc tgg aat atc ccc atg ggc ctg ctc ttc aac cag 1440
Asp Arg Thr Ala Gly Trp Asn Ile Pro Met Gly Leu Leu Phe Asn Gln
465                 470                 475                 480

acg ggc tcc tgc aaa ttt gat gaa tat ttc agt caa agc tgt gcc cct 1488
Thr Gly Ser Cys Lys Phe Asp Glu Tyr Phe Ser Gln Ser Cys Ala Pro
                485                 490                 495

ggg tct gac ccg aga tct aat ctc tgt gct ctg tgt aat ggc gac gag 1536
Gly Ser Asp Pro Arg Ser Asn Leu Cys Ala Leu Cys Asn Gly Asp Glu
            500                 505                 510

cag ggt gag aat aag tgc gtg ccc aac agc aac gag aga tac tac ggc 1584
Gln Gly Glu Asn Lys Cys Val Pro Asn Ser Asn Glu Arg Tyr Tyr Gly
        515                 520                 525

tac act ggg gct ttc cgg tgc ctg gct gag aat gct gga gac gtt gca 1632
Tyr Thr Gly Ala Phe Arg Cys Leu Ala Glu Asn Ala Gly Asp Val Ala
    530                 535                 540

ttt gtg aaa gat gtc act gtc ttg cag aac act gat gga aat aac aat 1680
Phe Val Lys Asp Val Thr Val Leu Gln Asn Thr Asp Gly Asn Asn Asn
545                 550                 555                 560

gag gca tgg gct aag gat ttg aag ctg gca gac ttt gcg ctg ctg tgc 1728
Glu Ala Trp Ala Lys Asp Leu Lys Leu Ala Asp Phe Ala Leu Leu Cys
                565                 570                 575

ctc gat ggc aaa cgg aag cct gtg act gag gct aga agc tgc cat ctt 1776
Leu Asp Gly Lys Arg Lys Pro Val Thr Glu Ala Arg Ser Cys His Leu
            580                 585                 590

gcc atg gcc ccg aat cat gcc gtg gtg tct cgg atg gat aag gtg gaa 1824
Ala Met Ala Pro Asn His Ala Val Val Ser Arg Met Asp Lys Val Glu
        595                 600                 605

cgc ctg aaa cag gtg ttg ctc cac caa cag gct aaa ttt ggg aga aat 1872
Arg Leu Lys Gln Val Leu Leu His Gln Gln Ala Lys Phe Gly Arg Asn
    610                 615                 620

gga tct gac tgc ccg gac aag ttt tgc tta ttc cag tct gaa acc aaa 1920
Gly Ser Asp Cys Pro Asp Lys Phe Cys Leu Phe Gln Ser Glu Thr Lys
625                 630                 635                 640

aac ctt ctg ttc aat gac aac act gag tgt ctg gcc aga ctc cat gga 1968
Asn Leu Leu Phe Asn Asp Asn Thr Glu Cys Leu Ala Arg Leu His Gly
                645                 650                 655

aaa aca aca tat gaa aaa tat ttg gga cca cag tat gtc gca ggc att 2016
Lys Thr Thr Tyr Glu Lys Tyr Leu Gly Pro Gln Tyr Val Ala Gly Ile
            660                 665                 670

act aat ctg aaa aag tgc tca acc tcc ccc ctc ctg gaa gcc tgt gaa 2064
Thr Asn Leu Lys Lys Cys Ser Thr Ser Pro Leu Leu Glu Ala Cys Glu
        675                 680                 685

ttc ctc agg aag taa 2079
Phe Leu Arg Lys
    690

<210> 14
<211> 692
<212> PRT
<213> Homo sapiens

<400> 14

Gly Arg Arg Arg Arg Ser Val Gln Trp Cys Ala Val Ser Gln Pro Glu
1               5                   10                  15

Ala Thr Lys Cys Phe Gln Trp Gln Arg Asn Met Arg Lys Val Arg Gly
            20                  25                  30

Pro Pro Val Ser Cys Ile Lys Arg Asp Ser Pro Ile Gln Cys Ile Gln
        35                  40                  45

Ala Ile Ala Glu Asn Arg Ala Asp Ala Val Thr Leu Asp Gly Gly Phe
    50                  55                  60

Ile Tyr Glu Ala Gly Leu Ala Pro Tyr Lys Leu Arg Pro Val Ala Ala
65                  70                  75                  80

Glu Val Tyr Gly Thr Glu Arg Gln Pro Arg Thr His Tyr Tyr Ala Val
                85                  90                  95

Ala Val Val Asn Lys Gly Gly Ser Phe Gln Leu Asn Glu Leu Gln Gly
            100                 105                 110

Leu Lys Ser Cys His Thr Gly Leu Arg Arg Thr Ala Gly Trp Asn Val
        115                 120                 125

Pro Ile Gly Thr Leu Arg Pro Phe Leu Asn Trp Thr Gly Pro Pro Glu
    130                 135                 140

Pro Ile Glu Ala Ala Val Ala Arg Phe Phe Ser Ala Ser Cys Val Pro
145                 150                 155                 160

Gly Ala Asp Lys Gly Gln Phe Pro Asn Leu Cys Arg Leu Cys Ala Gly
                165                 170                 175

Thr Gly Glu Asn Lys Cys Ala Phe Ser Ser Gln Glu Pro Tyr Phe Ser
            180                 185                 190

Tyr Ser Gly Ala Phe Lys Cys Leu Arg Asp Gly Ala Gly Asp Val Ala
        195                 200                 205

Phe Ile Arg Glu Ser Thr Val Phe Glu Asp Leu Ser Asp Glu Ala Glu
    210                 215                 220

Arg Asp Glu Tyr Glu Leu Leu Cys Pro Asp Asn Thr Arg Lys Pro Val
225                 230                 235                 240

Asp Lys Phe Lys Asp Cys His Leu Ala Arg Val Pro Ser His Ala Val
                245                 250                 255

Val Ala Arg Ser Val Asn Gly Lys Glu Asp Ala Ile Trp Asn Leu Leu
            260                 265                 270

Arg Gln Ala Gln Glu Lys Phe Gly Lys Asp Lys Ser Pro Lys Phe Gln
        275                 280                 285

Leu Phe Gly Ser Pro Ser Gly Gln Lys Asp Leu Leu Phe Lys Asp Ser
    290                 295                 300

Ala Ile Gly Phe Ser Arg Val Pro Pro Arg Ile Asp Ser Gly Leu Tyr
305                 310                 315                 320

Leu Gly Ser Gly Tyr Phe Thr Ala Ile Gln Asn Leu Arg Lys Ser Glu
                325                 330                 335

Glu Glu Val Ala Ala Arg Arg Ala Arg Val Val Trp Cys Ala Val Gly
            340                 345                 350

Glu Gln Glu Leu Arg Lys Cys Asn Gln Trp Ser Gly Leu Ser Glu Gly
        355                 360                 365

Ser Val Thr Cys Ser Ser Ala Ser Thr Thr Glu Asp Cys Ile Ala Leu
    370                 375                 380

Val Leu Lys Gly Glu Ala Asp Ala Met Ser Leu Asp Glu Gly Tyr Val
385                 390                 395                 400

Tyr Thr Ala Gly Lys Cys Gly Leu Val Pro Val Leu Ala Glu Asn Tyr
                405                 410                 415

Lys Ser Gln Gln Ser Ser Asp Pro Asp Pro Asn Cys Val Asp Arg Pro
            420                 425                 430

Val Glu Gly Tyr Leu Ala Val Ala Val Val Arg Arg Ser Asp Thr Ser
        435                 440                 445

Leu Thr Trp Asn Ser Val Lys Gly Lys Lys Ser Cys His Thr Ala Val
    450                 455                 460

Asp Arg Thr Ala Gly Trp Asn Ile Pro Met Gly Leu Leu Phe Asn Gln
465                 470                 475                 480

Thr Gly Ser Cys Lys Phe Asp Glu Tyr Phe Ser Gln Ser Cys Ala Pro
                485                 490                 495

Gly Ser Asp Pro Arg Ser Asn Leu Cys Ala Leu Cys Asn Gly Asp Glu
            500                 505                 510

Gln Gly Glu Asn Lys Cys Val Pro Asn Ser Asn Glu Arg Tyr Tyr Gly
        515                 520                 525

Tyr Thr Gly Ala Phe Arg Cys Leu Ala Glu Asn Ala Gly Asp Val Ala
    530                 535                 540

Phe Val Lys Asp Val Thr Val Leu Gln Asn Thr Asp Gly Asn Asn Asn
545                 550                 555                 560

Glu Ala Trp Ala Lys Asp Leu Lys Leu Ala Asp Phe Ala Leu Leu Cys
                565                 570                 575

Leu Asp Gly Lys Arg Lys Pro Val Thr Glu Ala Arg Ser Cys His Leu
            580                 585                 590

Ala Met Ala Pro Asn His Ala Val Val Ser Arg Met Asp Lys Val Glu
        595                 600                 605

Arg Leu Lys Gln Val Leu Leu His Gln Gln Ala Lys Phe Gly Arg Asn
    610                 615                 620

Gly Ser Asp Cys Pro Asp Lys Phe Cys Leu Phe Gln Ser Glu Thr Lys
625                 630                 635                 640

Asn Leu Leu Phe Asn Asp Asn Thr Glu Cys Leu Ala Arg Leu His Gly
                645                 650                 655

Lys Thr Thr Tyr Glu Lys Tyr Leu Gly Pro Gln Tyr Val Ala Gly Ile
            660                 665                 670

Thr Asn Leu Lys Lys Cys Ser Thr Ser Pro Leu Leu Glu Ala Cys Glu
        675                 680                 685

Phe Leu Arg Lys
    690

<210>  15
<211>  692
<212>  PRT
<213>  Homo sapiens

<400>  15

Gly Arg Arg Arg Arg Ser Val Gln Trp Cys Ala Val Ser Gln Pro Glu
1               5                   10                  15

Ala Thr Lys Cys Phe Gln Trp Gln Arg Asn Met Arg Lys Val Arg Gly
            20                  25                  30

Pro Pro Val Ser Cys Ile Lys Arg Asp Ser Pro Ile Gln Cys Ile Gln
        35                  40                  45

Ala Ile Ala Glu Asn Arg Ala Asp Ala Val Thr Leu Asp Gly Gly Phe
    50                  55                  60

Ile Tyr Glu Ala Gly Leu Ala Pro Tyr Lys Leu Arg Pro Val Ala Ala
65                  70                  75                  80

Glu Val Tyr Gly Thr Glu Arg Gln Pro Arg Thr His Tyr Tyr Ala Val
                85                  90                  95

Ala Val Val Asn Lys Gly Gly Ser Phe Gln Leu Asn Glu Leu Gln Gly
            100                 105                 110

Leu Lys Ser Cys His Thr Gly Leu Arg Arg Thr Ala Gly Trp Asn Val
        115                 120                 125

Pro Ile Gly Thr Leu Arg Pro Phe Leu Asn Trp Thr Gly Pro Pro Glu
    130                 135                 140

Pro Ile Glu Ala Ala Val Ala Arg Phe Phe Ser Ala Ser Cys Val Pro
145                 150                 155                 160

Gly Ala Asp Lys Gly Gln Phe Pro Asn Leu Cys Arg Leu Cys Ala Gly
                165                 170                 175

Thr Gly Glu Asn Lys Cys Ala Phe Ser Ser Gln Glu Pro Tyr Phe Ser
            180                 185                 190

Tyr Ser Gly Ala Phe Lys Cys Leu Arg Asp Gly Ala Gly Asp Val Ala
        195                 200                 205

Phe Ile Arg Glu Ser Thr Val Phe Glu Asp Leu Ser Asp Glu Ala Glu
    210                 215                 220

Arg Asp Glu Tyr Glu Leu Leu Cys Pro Asp Asn Thr Arg Lys Pro Val
225                 230                 235                 240

Asp Lys Phe Lys Asp Cys His Leu Ala Arg Val Pro Ser His Ala Val
                245                 250                 255

Val Ala Arg Ser Val Asn Gly Lys Glu Asp Ala Ile Trp Asn Leu Leu
            260                 265                 270

Arg Gln Ala Gln Glu Lys Phe Gly Lys Asp Lys Ser Pro Lys Phe Gln
        275                 280                 285

Leu Phe Gly Ser Pro Ser Gly Gln Lys Asp Leu Leu Phe Lys Asp Ser
    290                 295                 300

Ala Ile Gly Phe Ser Arg Val Pro Pro Arg Ile Asp Ser Gly Leu Tyr
305                 310                 315                 320

Leu Gly Ser Gly Tyr Phe Thr Ala Ile Gln Asn Leu Arg Lys Ser Glu
                325                 330                 335

Glu Glu Val Ala Ala Arg Arg Ala Arg Val Val Trp Cys Ala Val Gly
            340                 345                 350

Glu Gln Glu Leu Arg Lys Cys Asn Gln Trp Ser Gly Leu Ser Glu Gly
        355                 360                 365

Ser Val Thr Cys Ser Ser Ala Ser Thr Thr Glu Asp Cys Ile Ala Leu
    370                 375                 380

Val Leu Lys Gly Glu Ala Asp Ala Met Ser Leu Asp Glu Gly Tyr Val
385                 390                 395                 400

Tyr Thr Ala Gly Lys Cys Gly Leu Val Pro Val Leu Ala Glu Asn Tyr
                405                 410                 415

Lys Ser Gln Gln Ser Ser Asp Pro Asp Pro Asn Cys Val Asp Arg Pro
            420                 425                 430

Val Glu Gly Tyr Leu Ala Val Ala Val Val Arg Arg Ser Asp Thr Ser
        435                 440                 445

Leu Thr Trp Asn Ser Val Lys Gly Lys Lys Ser Cys His Thr Ala Val
    450                 455                 460

Asp Arg Thr Ala Gly Trp Asn Ile Pro Met Gly Leu Leu Phe Asn Gln
465                 470                 475                 480

Thr Gly Ser Cys Lys Phe Asp Glu Tyr Phe Ser Gln Ser Cys Ala Pro
                485                 490                 495

Gly Ser Asp Pro Arg Ser Asn Leu Cys Ala Leu Cys Asn Gly Asp Glu
            500                 505                 510

Gln Gly Glu Asn Lys Cys Val Pro Asn Ser Asn Glu Arg Tyr Tyr Gly
        515                 520                 525

Tyr Thr Gly Ala Phe Arg Cys Leu Ala Glu Asn Ala Gly Asp Val Ala
    530                 535                 540

Phe Val Lys Asp Val Thr Val Leu Gln Asn Thr Asp Gly Asn Asn Asn
545                 550                 555                 560

Glu Ala Trp Ala Lys Asp Leu Lys Leu Ala Asp Phe Ala Leu Leu Cys
                565                 570                 575

Leu Asp Gly Lys Arg Lys Pro Val Thr Glu Ala Arg Ser Cys His Leu
            580                 585                 590

Ala Met Ala Pro Asn His Ala Val Val Ser Arg Met Asp Lys Val Glu
        595                 600                 605

Arg Leu Lys Gln Val Leu Leu His Gln Gln Ala Lys Phe Gly Arg Asn
    610                 615                 620

Gly Ser Asp Cys Pro Asp Lys Phe Cys Leu Phe Gln Ser Glu Thr Lys
625                 630                 635                 640

Asn Leu Leu Phe Asn Asp Asn Thr Glu Cys Leu Ala Arg Leu His Gly
                645                 650                 655

Lys Thr Thr Tyr Glu Lys Tyr Leu Gly Pro Gln Tyr Val Ala Gly Ile
            660                 665                 670

Thr Asn Leu Lys Lys Cys Ser Thr Ser Pro Leu Leu Glu Ala Cys Glu
        675                 680                 685

Phe Leu Arg Lys
    690

<210>  16
<211>  2079
<212>  DNA
<213>  Artificial

<220>
<223>  Synthetic codon optimized human lactoferrin gene sequence

<400> 16
gggcggcggc ggcgctcggt gcagtggtgc gccgtgtccc agcccgaggc gaccaagtgc 60
ttccagtggc agcgcaacat gcggaaggtg cgcggcccgc cggtcagctg catcaagcgg 120
gactccccca tccaatgcat ccaggccatc gcggagaacc gcgccgacgc ggtcaccctg 180
gacggcgggt tcatctacga ggcggggctc gccccgtaca agctccgccc ggtggcggcg 240
gaggtgtacg gcaccgagcg ccagccgcgc acgcactact acgcggtggc cgtcgtcaag 300
aagggcgggt ccttccagct caacgagctg cagggcctga agtcgtgcca cacgggcctc 360
cggcggacgg cgggctggaa cgtgcccatc ggcaccctgc gccccttcct gaactggacc 420
ggcccgccgg agccgatcga ggccgccgtg gcccgcttct tcagcgcctc ctgcgtcccc 480
ggcgccgaca agggccagtt cccgaacctc tgccggctct gcgccgggac gggcgagaac 540
aagtgcgcct tctcctcgca ggagccgtac ttctcctact cgggcgcgtt caagtgcctc 600
cgcgacgggg ccggcgacgt ggcgttcatc cgcgagtcca ccgtgttcga ggacctctcc 660
gacgaggcgg agcgggacga gtacgagctg ctgtgccccg acaacacccg caagccggtg 720
gacaagttca aggactgcca cctggcgcgg gtgccctcgc acgcggtcgt cgcccgcagc 780
gtcaacggca aggaggacgc gatctggaac ctcctccgcc aggcccagga gaagttcggc 840
aaggacaagt cccccaagtt ccagctcttc gggagcccca gcggccagaa ggacctcctc 900
ttcaaggact ccgcgatcgg cttctcccgc gtccccccgc gcatcgactc cggcctgtac 960
ctcggctccg ggtacttcac cgcgatccag aacctccgga agagcgagga ggaggtggcg 1020
gcgcggcggg cccgcgtcgt gtggtgcgcc gtgggcgagc aggagctgcg gaagtgcaac 1080
cagtggagcg gcctgagcga ggggtcggtg acctgctcgt ccgccagcac caccgaggac 1140
tgcatcgcgc tcgtcctcaa gggggaggcc gacgcgatga gcctcgacgg ggggtacgtc 1200
tacaccgccg gcaagtgcgg cctggtcccg gtcctggcgg agaactacaa gtcgcagcag 1260
tccagcgacc ccgacccgaa ctgcgtggac cgccccgtcg agggctacct cgccgtggcc 1320
gtcgtgcgcc ggtccgacac ctccctgacg tggaacagcg tcaagggcaa gaagagctgc 1380
cacaccgccg tggaccgcac cgccggctgg aacatcccga tgggcctcct cttcaaccag 1440
accggctcct gcaagttcga cgagtacttc tcccagtcct gcgcccccgg ctcggacccc 1500
cgctccaacc tgtgcgccct ctgcatcggg gacgagcagg gcgagaacaa gtgcgtgccc 1560
aacagcaacg agcggtacta cggctacacg ggggccttcc gctgcctggc ggagaacgcc 1620
ggggacgtcg cgttcgtgaa ggacgtgacc gtgctgcaaa acacggacgg gaacaacaac 1680
gaggcgtggg cgaaggacct caagctcgcc gacttcgccc tgctgtgcct cgacggcaag 1740
cgcaagcccg tcaccgaggc gcggtcctgc cacctggcga tggcccccaa ccacgccgtc 1800
gtctcccgca tggacaaggt cgagcgcctc aagcaggtgc tcctgcacca gcaggccaag 1860
ttcggccgga acggcagcga ctgcccggac aagttctgcc tgttccagtc ggagaccaag 1920
aacctcctct tcaacgacaa caccgagtgc ctggcgcgcc tccacggcaa gaccacctac 1980
gagaagtacc tcggcccgca gtacgtcgcc ggcatcacca acctcaagaa gtgctccacc 2040

tcccccctcc tggaggcgtg cgagttcctc cgcaagtga                                2079

<210>  17
<211>  1830
<212>  DNA
<213>  Artificial

<220>
<223>  Synthetic fusion Gt1 SP/optimized HSA sequence

<220>
<221>  CDS
<222>  (1)..(1827)

<400>  17
atg gca tcc ata aat cgc ccc ata gtt ttc ttc aca gtt tgc ttg ttc        48
Met Ala Ser Ile Asn Arg Pro Ile Val Phe Phe Thr Val Cys Leu Phe
1               5                   10                  15

ctc ttg tgc gat ggc tcc cta gcc gac gcc cac aag agc gag gtg gcc        96
Leu Leu Cys Asp Gly Ser Leu Ala Asp Ala His Lys Ser Glu Val Ala
            20                  25                  30

cac cgc ttc aag gac ctc ggc gag gag aac ttc aag gcc ctc gtg ctc       144
His Arg Phe Lys Asp Leu Gly Glu Glu Asn Phe Lys Ala Leu Val Leu
        35                  40                  45

atc gcc ttc gcc cag tac ctc cag cag tgc ccg ttc gag gac cac gtg       192
Ile Ala Phe Ala Gln Tyr Leu Gln Gln Cys Pro Phe Glu Asp His Val
    50                  55                  60

aag ctc gtg aac gag gtg acc gag ttc gcc aag acc tgc gtg gcc gac       240
Lys Leu Val Asn Glu Val Thr Glu Phe Ala Lys Thr Cys Val Ala Asp
65                  70                  75                  80

gag agc gcc gag aac tgc gac aag agc ctc cac acc ctc ttc ggc gac       288
Glu Ser Ala Glu Asn Cys Asp Lys Ser Leu His Thr Leu Phe Gly Asp
                85                  90                  95

aag ctc tgc acc gtg gcc acc ctc cgc gag acc tac ggc gag atg gcc       336
Lys Leu Cys Thr Val Ala Thr Leu Arg Glu Thr Tyr Gly Glu Met Ala
            100                 105                 110

gac tgc tgc gcc aag cag gag ccg gag cgc aac gag tgc ttc ctc gag       384
Asp Cys Cys Ala Lys Gln Glu Pro Glu Arg Asn Glu Cys Phe Leu Glu
        115                 120                 125

cac aag gac gac acc ccg aac ctc ccg cgc ctc gtg cgc ccg gag gtg       432
His Lys Asp Asp Thr Pro Asn Leu Pro Arg Leu Val Arg Pro Glu Val
    130                 135                 140

gac gtg atg tgc acc gcc ttc cac gac aac gag gag acc ttc ctc aag       480
Asp Val Met Cys Thr Ala Phe His Asp Asn Glu Glu Thr Phe Leu Lys
145                 150                 155                 160

aag tac ctc tac gag atc gcc cgc cgc cac ccg tac ttc tac gcc ccg       528
Lys Tyr Leu Tyr Glu Ile Ala Arg Arg His Pro Tyr Phe Tyr Ala Pro
                165                 170                 175

gag ctc ctc ttc ttc gcc aag cgc tac aag gcc gcc ttc acc gag tgc       576
Glu Leu Leu Phe Phe Ala Lys Arg Tyr Lys Ala Ala Phe Thr Glu Cys
            180                 185                 190

tgc cag gcc gcc gac aag gcc gcc tgc ctc ctc ccg aag ctc gac gag       624
Cys Gln Ala Ala Asp Lys Ala Ala Cys Leu Leu Pro Lys Leu Asp Glu

        195                 200                 205

ctc cgc gac gag ggc aag gcc tcc agc gcc aag cag cgc ctc aag tgc 672
Leu Arg Asp Glu Gly Lys Ala Ser Ser Ala Lys Gln Arg Leu Lys Cys
    210                 215                 220

gcc agc ctc cag aag ttc ggc gag cgc gcc ttc aag gcc tgg gcc gtg 720
Ala Ser Leu Gln Lys Phe Gly Glu Arg Ala Phe Lys Ala Trp Ala Val
225                 230                 235                 240

gcc cgc ctc agc cag cgc ttc ccg aag gcc gag ttc gcc gag gtg tcc 768
Ala Arg Leu Ser Gln Arg Phe Pro Lys Ala Glu Phe Ala Glu Val Ser
                245                 250                 255

aag ctc gtg acc gac ctc acc aag gtg cac acc gag tgc tgc cac ggc 816
Lys Leu Val Thr Asp Leu Thr Lys Val His Thr Glu Cys Cys His Gly
            260                 265                 270

gac ctc ctg gag tgc gcc gac gac cgc gcc gac ctc gcc aag tac atc 864
Asp Leu Leu Glu Cys Ala Asp Asp Arg Ala Asp Leu Ala Lys Tyr Ile
        275                 280                 285

tgc gag aac cag gac agc atc tcc agc aag ctc aag gag tgc tgc gag 912
Cys Glu Asn Gln Asp Ser Ile Ser Ser Lys Leu Lys Glu Cys Cys Glu
    290                 295                 300

aag ccg ctc ctg gag aag tcc cac tgc atc gcc gag gtg gag aac gac 960
Lys Pro Leu Leu Glu Lys Ser His Cys Ile Ala Glu Val Glu Asn Asp
305                 310                 315                 320

gag atg ccg gcc gac ctc ccg tcc ctc gcc gcc gac ttc gtg gag agc 1008
Glu Met Pro Ala Asp Leu Pro Ser Leu Ala Ala Asp Phe Val Glu Ser
                325                 330                 335

aag gac gtg tgc aag aac tac gcc gag gcc aag gac gtc ttc ctc ggc 1056
Lys Asp Val Cys Lys Asn Tyr Ala Glu Ala Lys Asp Val Phe Leu Gly
            340                 345                 350

atg ttc ctc tac gag tac gcc cgc cgc cac ccg gac tac tcc gtg gtg 1104
Met Phe Leu Tyr Glu Tyr Ala Arg Arg His Pro Asp Tyr Ser Val Val
        355                 360                 365

ctc ctc ctc cgc ctc gcc aag acc tac gag acc acc ctg gag aag tgc 1152
Leu Leu Leu Arg Leu Ala Lys Thr Tyr Glu Thr Thr Leu Glu Lys Cys
    370                 375                 380

tgc gcc gcc gcc gac ccg cac gag tgc tac gcc aag gtg ttc gac gag 1200
Cys Ala Ala Ala Asp Pro His Glu Cys Tyr Ala Lys Val Phe Asp Glu
385                 390                 395                 400

ttc aag ccg ctc gtg gag gag ccg cag aac ctc atc aag cag aac tgc 1248
Phe Lys Pro Leu Val Glu Glu Pro Gln Asn Leu Ile Lys Gln Asn Cys
                405                 410                 415

gag ctc ttc gag cag ctc ggc gag tac aag ttc cag aac gcc ctc ctc 1296
Glu Leu Phe Glu Gln Leu Gly Glu Tyr Lys Phe Gln Asn Ala Leu Leu
            420                 425                 430

gtg cgc tac acc aag aag gtg ccg cag gtg tcc acc ccg acc ctc gtg 1344
Val Arg Tyr Thr Lys Lys Val Pro Gln Val Ser Thr Pro Thr Leu Val
        435                 440                 445

gag gtg tcc cgc aac ctc ggc aag gtg ggc agc aag tgc tgc aag cac 1392
Glu Val Ser Arg Asn Leu Gly Lys Val Gly Ser Lys Cys Cys Lys His
    450                 455                 460

ccg gag gcc aag cgc atg ccg tgc gcc gag gac tac ctc tcc gtg gtg 1440
Pro Glu Ala Lys Arg Met Pro Cys Ala Glu Asp Tyr Leu Ser Val Val

465                 470                 475                 480

ctc aac cag ctc tgc gtg ctc cac gag aag acc ccg gtg agc gac cgc       1488
Leu Asn Gln Leu Cys Val Leu His Glu Lys Thr Pro Val Ser Asp Arg
                485                 490                 495

gtg acc aag tgc tgc acc gag agc ctc gtg aac cgc cgc ccg tgc ttc       1536
Val Thr Lys Cys Cys Thr Glu Ser Leu Val Asn Arg Arg Pro Cys Phe
            500                 505                 510

tcc gcc ctg gag gtc gac gag acc tac gtc ccg aag gag ttc aac gcc       1584
Ser Ala Leu Glu Val Asp Glu Thr Tyr Val Pro Lys Glu Phe Asn Ala
        515                 520                 525

gag acc ttc acc ttc cac gcc gac atc tgc acc ctc tcc gag aag gag       1632
Glu Thr Phe Thr Phe His Ala Asp Ile Cys Thr Leu Ser Glu Lys Glu
    530                 535                 540

cgc cag atc aag aag cag acc gcc ctc gtc gag ctc gtg aag cac aag       1680
Arg Gln Ile Lys Lys Gln Thr Ala Leu Val Glu Leu Val Lys His Lys
545                 550                 555                 560

ccg aag gcc acc aag gag cag ctc aag gcc gtg atg gac gac ttc gcc       1728
Pro Lys Ala Thr Lys Glu Gln Leu Lys Ala Val Met Asp Asp Phe Ala
                565                 570                 575

gcc ttc gtg gag aag tgc tgc aag gcc gac gac aag gag acc tgc ttc       1776
Ala Phe Val Glu Lys Cys Cys Lys Ala Asp Asp Lys Glu Thr Cys Phe
            580                 585                 590

gcc gag gag ggc aag aag ctc gtg gcc gcc agc cag gcc gcc ctc ggc       1824
Ala Glu Glu Gly Lys Lys Leu Val Ala Ala Ser Gln Ala Ala Leu Gly
        595                 600                 605

ctc tga                                                               1830
Leu

<210>  18
<211>  609
<212>  PRT
<213>  Artificial

<220>
<223>  Synthetic Construct

<400>  18

Met Ala Ser Ile Asn Arg Pro Ile Val Phe Phe Thr Val Cys Leu Phe
1               5                   10                  15

Leu Leu Cys Asp Gly Ser Leu Ala Asp Ala His Lys Ser Glu Val Ala
            20                  25                  30

His Arg Phe Lys Asp Leu Gly Glu Glu Asn Phe Lys Ala Leu Val Leu
        35                  40                  45

Ile Ala Phe Ala Gln Tyr Leu Gln Gln Cys Pro Phe Glu Asp His Val
    50                  55                  60

Lys Leu Val Asn Glu Val Thr Glu Phe Ala Lys Thr Cys Val Ala Asp
65                  70                  75                  80

Glu Ser Ala Glu Asn Cys Asp Lys Ser Leu His Thr Leu Phe Gly Asp
                85                  90                  95

Lys Leu Cys Thr Val Ala Thr Leu Arg Glu Thr Tyr Gly Glu Met Ala
            100                 105                 110

Asp Cys Cys Ala Lys Gln Glu Pro Glu Arg Asn Glu Cys Phe Leu Glu
        115                 120                 125

His Lys Asp Asp Thr Pro Asn Leu Pro Arg Leu Val Arg Pro Glu Val
    130                 135                 140

Asp Val Met Cys Thr Ala Phe His Asp Asn Glu Glu Thr Phe Leu Lys
145                 150                 155                 160

Lys Tyr Leu Tyr Glu Ile Ala Arg Arg His Pro Tyr Phe Tyr Ala Pro
                165                 170                 175

Glu Leu Leu Phe Phe Ala Lys Arg Tyr Lys Ala Ala Phe Thr Glu Cys
            180                 185                 190

Cys Gln Ala Ala Asp Lys Ala Ala Cys Leu Leu Pro Lys Leu Asp Glu
        195                 200                 205

Leu Arg Asp Glu Gly Lys Ala Ser Ser Ala Lys Gln Arg Leu Lys Cys
    210                 215                 220

Ala Ser Leu Gln Lys Phe Gly Glu Arg Ala Phe Lys Ala Trp Ala Val
225                 230                 235                 240

Ala Arg Leu Ser Gln Arg Phe Pro Lys Ala Glu Phe Ala Glu Val Ser
                245                 250                 255

Lys Leu Val Thr Asp Leu Thr Lys Val His Thr Glu Cys Cys His Gly
            260                 265                 270

Asp Leu Leu Glu Cys Ala Asp Asp Arg Ala Asp Leu Ala Lys Tyr Ile
        275                 280                 285

Cys Glu Asn Gln Asp Ser Ile Ser Ser Lys Leu Lys Glu Cys Cys Glu
    290                 295                 300

Lys Pro Leu Leu Glu Lys Ser His Cys Ile Ala Glu Val Glu Asn Asp
305                 310                 315                 320

Glu Met Pro Ala Asp Leu Pro Ser Leu Ala Ala Asp Phe Val Glu Ser
                325                 330                 335

Lys Asp Val Cys Lys Asn Tyr Ala Glu Ala Lys Asp Val Phe Leu Gly
            340                 345                 350

Met Phe Leu Tyr Glu Tyr Ala Arg Arg His Pro Asp Tyr Ser Val Val
        355                 360                 365

Leu Leu Leu Arg Leu Ala Lys Thr Tyr Glu Thr Thr Leu Glu Lys Cys
    370                 375                 380

Cys Ala Ala Ala Asp Pro His Glu Cys Tyr Ala Lys Val Phe Asp Glu
385                 390                 395                 400

Phe Lys Pro Leu Val Glu Glu Pro Gln Asn Leu Ile Lys Gln Asn Cys
                405                 410                 415

Glu Leu Phe Glu Gln Leu Gly Glu Tyr Lys Phe Gln Asn Ala Leu Leu
            420                 425                 430

Val Arg Tyr Thr Lys Lys Val Pro Gln Val Ser Thr Pro Thr Leu Val
        435                 440                 445

Glu Val Ser Arg Asn Leu Gly Lys Val Gly Ser Lys Cys Cys Lys His
    450                 455                 460

Pro Glu Ala Lys Arg Met Pro Cys Ala Glu Asp Tyr Leu Ser Val Val
465                 470                 475                 480

Leu Asn Gln Leu Cys Val Leu His Glu Lys Thr Pro Val Ser Asp Arg
                485                 490                 495

Val Thr Lys Cys Cys Thr Glu Ser Leu Val Asn Arg Arg Pro Cys Phe
            500                 505                 510

Ser Ala Leu Glu Val Asp Glu Thr Tyr Val Pro Lys Glu Phe Asn Ala
        515                 520                 525

Glu Thr Phe Thr Phe His Ala Asp Ile Cys Thr Leu Ser Glu Lys Glu
    530                 535                 540

Arg Gln Ile Lys Lys Gln Thr Ala Leu Val Glu Leu Val Lys His Lys
545                 550                 555                 560

Pro Lys Ala Thr Lys Glu Gln Leu Lys Ala Val Met Asp Asp Phe Ala
                565                 570                 575

Ala Phe Val Glu Lys Cys Cys Lys Ala Asp Asp Lys Glu Thr Cys Phe
            580                 585                 590

Ala Glu Glu Gly Lys Lys Leu Val Ala Ala Ser Gln Ala Ala Leu Gly
        595                 600                 605

Leu

<210> 19
<211> 609
<212> PRT
<213> Artificial

<220>
<223> Synthetic fusion Gt1 SP/optimized HSA sequence

<400> 19

Met Ala Ser Ile Asn Arg Pro Ile Val Phe Phe Thr Val Cys Leu Phe
1               5                   10                  15

Leu Leu Cys Asp Gly Ser Leu Ala Asp Ala His Lys Ser Glu Val Ala
            20                  25                  30

His Arg Phe Lys Asp Leu Gly Glu Glu Asn Phe Lys Ala Leu Val Leu
        35                  40                  45

Ile Ala Phe Ala Gln Tyr Leu Gln Gln Cys Pro Phe Glu Asp His Val
    50                  55                  60

Lys Leu Val Asn Glu Val Thr Glu Phe Ala Lys Thr Cys Val Ala Asp
65                  70                  75                  80

Glu Ser Ala Glu Asn Cys Asp Lys Ser Leu His Thr Leu Phe Gly Asp
                85                  90                  95

Lys Leu Cys Thr Val Ala Thr Leu Arg Glu Thr Tyr Gly Glu Met Ala
            100                 105                 110

Asp Cys Cys Ala Lys Gln Glu Pro Glu Arg Asn Glu Cys Phe Leu Glu
        115                 120                 125

His Lys Asp Asp Thr Pro Asn Leu Pro Arg Leu Val Arg Pro Glu Val
    130                 135                 140

Asp Val Met Cys Thr Ala Phe His Asp Asn Glu Glu Thr Phe Leu Lys
145                 150                 155                 160

Lys Tyr Leu Tyr Glu Ile Ala Arg Arg His Pro Tyr Phe Tyr Ala Pro
                165                 170                 175

Glu Leu Leu Phe Phe Ala Lys Arg Tyr Lys Ala Ala Phe Thr Glu Cys
            180                 185                 190

Cys Gln Ala Ala Asp Lys Ala Ala Cys Leu Leu Pro Lys Leu Asp Glu
        195                 200                 205

Leu Arg Asp Glu Gly Lys Ala Ser Ser Ala Lys Gln Arg Leu Lys Cys
    210                 215                 220

Ala Ser Leu Gln Lys Phe Gly Glu Arg Ala Phe Lys Ala Trp Ala Val

225                 230                 235                 240

Ala Arg Leu Ser Gln Arg Phe Pro Lys Ala Glu Phe Ala Glu Val Ser
                245                 250                 255

Lys Leu Val Thr Asp Leu Thr Lys Val His Thr Glu Cys Cys His Gly
            260                 265                 270

Asp Leu Leu Glu Cys Ala Asp Asp Arg Ala Asp Leu Ala Lys Tyr Ile
        275                 280                 285

Cys Glu Asn Gln Asp Ser Ile Ser Ser Lys Leu Lys Glu Cys Cys Glu
    290                 295                 300

Lys Pro Leu Leu Glu Lys Ser His Cys Ile Ala Glu Val Glu Asn Asp
305                 310                 315                 320

Glu Met Pro Ala Asp Leu Pro Ser Leu Ala Ala Asp Phe Val Glu Ser
                325                 330                 335

Lys Asp Val Cys Lys Asn Tyr Ala Glu Ala Lys Asp Val Phe Leu Gly
            340                 345                 350

Met Phe Leu Tyr Glu Tyr Ala Arg Arg His Pro Asp Tyr Ser Val Val
        355                 360                 365

Leu Leu Leu Arg Leu Ala Lys Thr Tyr Glu Thr Thr Leu Glu Lys Cys
    370                 375                 380

Cys Ala Ala Ala Asp Pro His Glu Cys Tyr Ala Lys Val Phe Asp Glu
385                 390                 395                 400

Phe Lys Pro Leu Val Glu Glu Pro Gln Asn Leu Ile Lys Gln Asn Cys
                405                 410                 415

Glu Leu Phe Glu Gln Leu Gly Glu Tyr Lys Phe Gln Asn Ala Leu Leu
            420                 425                 430

Val Arg Tyr Thr Lys Lys Val Pro Gln Val Ser Thr Pro Thr Leu Val
        435                 440                 445

Glu Val Ser Arg Asn Leu Gly Lys Val Gly Ser Lys Cys Cys Lys His
    450                 455                 460

Pro Glu Ala Lys Arg Met Pro Cys Ala Glu Asp Tyr Leu Ser Val Val
465                 470                 475                 480

Leu Asn Gln Leu Cys Val Leu His Glu Lys Thr Pro Val Ser Asp Arg
                485                 490                 495

Val Thr Lys Cys Cys Thr Glu Ser Leu Val Asn Arg Arg Pro Cys Phe

500                 505                 510

Ser Ala Leu Glu Val Asp Glu Thr Tyr Val Pro Lys Glu Phe Asn Ala
        515                 520                 525

Glu Thr Phe Thr Phe His Ala Asp Ile Cys Thr Leu Ser Glu Lys Glu
    530                 535                 540

Arg Gln Ile Lys Lys Gln Thr Ala Leu Val Glu Leu Val Lys His Lys
545                 550                 555                 560

Pro Lys Ala Thr Lys Glu Gln Leu Lys Ala Val Met Asp Asp Phe Ala
                565                 570                 575

Ala Phe Val Glu Lys Cys Cys Lys Ala Asp Asp Lys Glu Thr Cys Phe
            580                 585                 590

Ala Glu Glu Gly Lys Lys Leu Val Ala Ala Ser Gln Ala Ala Leu Gly
        595                 600                 605

Leu

## Description

This application claims priority benefit to U.S. provisional application Serial No. 60/694,236 filed June 28, 2006. The priority application is incorporated herein by reference in its entirety.

### 1. Field of the Invention:

The present invention relates to the production and use of recombinant proteins produced by plant cells as components of cell culture media.

### 2. Background of the Invention:

Cell culture techniques allow animal or plant cells that are removed from tissues to grow when supplied with the appropriate nutrients and conditions. The cells are capable of dividing and can continue to grow until limited by some culture variables such as nutrient depletion or toxic buildup (Butler, M. & Jenkins, H., Nutritional aspects of growth of animal cells in culture, J of Biotechnol. (1989) 12: 97-110). Cell culture techniques have a number of applications including investigation of the normal physiology or biochemistry of cells (Balaban, B. & Urman, B., Embryo culture as a diagnostic tool, Reprod. Biomed. Online (2003) 7(6): 671-82), testing the effect of various chemical compounds or drugs on specific cell types (Farkas, D. & Tannenbaum, S.R., In vitro methods to study chemically-induced hepatotoxicity: a literature review, Curr. Drug Metab. (2005) 6(2): 111-25), studying the sequential or parallel combination of various cell types to generate artificial tissues (Wang et al., Cartilage tissue engineering with silk scaffolds and human articular chondrocytes, Biomaterials (2006)), and synthesizing valuable biologics from large scale cell cultures (Zeilinger et al., Three-dimensional do-culture of primary human liver cells in bioreactors for in vitro drug studies: effects of the initial cell quality on the long-term maintenance of hepatocytespecific functions, Altern. Lab Anim. (2002) 30(5): 525-38). Cell culture techniques have also been used for in vitro fertilization (Blake et al., Protein supplementation of human IVF culture media, J Assist. Reprod. Genet. (2002) 19(3): 137-43; Bungum et al., Recombinant human albumin as protein source in culture media used for IVF: a prospective randomized study, Reprod. Biomed. Online (2002) 4(3): 233-6), stem cell research (Conley et al., Derivation, propagation and differentiation of human embryonic stem cells, Int. J Biochem. Cell Biol. (2004) 36(4): 555-67), vaccine production (Chuang et al., Pharmaceutical strategies utilizing recombinant human serum albumin, Pharm. Res. (2002) 19(5): 569-77; GlaxoSmithKline, Publisher. HAVRIX^{®} (Hepatitis A Vaccine, Inactivated) - Prescribing Information (2005), available at http://us.gsk.com/products/assets/us_havrix.pdf; Innis et al., Protection against hepatitis A by an inactivated vaccine, JAMA (1994) 271(17): 1328-34; Merck, Publisher. PROQUAD^{®} - Measles, Mumps, Rubella, and Varicella (Oka/Merck) Virus Vaccine Live - Prescribing Information (2005), available at http://www.merck.com/product/usa/pi_circulars/p/proquad/proquad_pl.pdf; Litwin, J., The growth of Vero cells In suspension as cell-aggregates in serum-free media, Cytotechnology (1992) 10(2): 169-74), tissue engineering including artificial skin (Atala, A., Future perspectives in reconstructive surgery using tissue engineering, Urol. Clin. North Am. (1999) 26(1): 157-65, ix-x; Sher, et al., Targeting perlecan in human keratinocytes reveals novel roles for perlecan in epidermal formation, J Biol. Chem. (2006) 281(8): 5178-87) and organs (Neronov et al., Integrity of endothelium in cryopreserved human cornea, Cryo Letters (2005) 26(2): 131-6; Han, et al., Interleukin-1alpha-induced proteolytic activation of metalloproteinase-9 by human skin, Surgery (2005) 138(5): 932-9) and gene and cell therapy (Chadd, H.E. & Chamow, S.M., Therapeutic antibody expression technology, Curr. Opin. Biotechnol. (2001) 12(2): 188-94).

The biologics encompass a broad range of cell products and include specific proteins or viruses that require animal cells for propagation. For example, therapeutic proteins such as monoclonal antibodies can be synthesized in large quantities by growing genetically engineered cells in large-scale cultures (Dewar et al., Industrial implementation of in vitro production of monoclonal antibodies, llar J (2005) 46(3): 307-13). The number of such commercially valuable biologics has increased rapidly over the last decade and has led to the present widespread interest in mammalian cell culture technology (Mizrahi, A., Biologicals produced from animal cells in culture-an overview, Biotechnol. Adv. (1988) 6(2): 207-20).

The major advantage of using cell culture for any of the above applications is the consistency and reproducibility of results that can be obtained from using a batch of clonal cells. The need for cell culture, especially at large scale, became apparent with the need for viral vaccines. Major epidemics of polio in the 1940s and 1950s promoted a lot of effort to develop an effective vaccine. In 1949, it was shown that poliovirus could be grown in cultures of human cells, which led to considerable interest in the development of large quantities of the polio vaccine using cell culture (Ligon, B.L., Thomas Huckle Weller MD: Nobel Laureate and research pioneer in poliomyelitis, varicella-zoster virus, cytomegalovirus, rubella, and other infectious diseases, Semin. Pediatr. Infect. Dis. (2002) 13(1): 55-63). The polio vaccine, produced from deactivated virus, became one of the first commercial products of cultured animal cells (Furesz, J., Developments in the production and quality control of poliovirus vaccines - Historical perspectives, Biologicals (2006)).

Many components of cell culture media are obtained from animal sources, including lactoferrin, transferrin and serum albumin. These components are extracted from animal sources, purified, and then combined with other inorganic ingredients to constitute cell culture media (Mizrahi, A. & Lazar, A., Media for cultivation of animal cells: an overview, Cytotechnology (1988) 1: 199-214). Cell culture components produced in this way have several undesirable features. For one, there is no way to ensure that extraneous animal products are completely excluded from the component, no matter how precise the purification techniques. Animal-derived components can carry the risk of contamination of pathogens of animal and human origin such as BSE, HIV and hepatitis virus (Chamberland et al., Emerging infectious disease issues in blood safety, Emerg. Infect. Dis. (2001) 7(3 Suppl): 552-3; Hepatitis C: A Brief Review, America's Blood Centers (October 1998) vol. 1, issue 3 (Strong, M. ed.), available at http://www.americasblood.org/index.cfm?fuseaction=display.showPage&pageID=133; Weinberg et al., Legal, financial, and public health consequences of HIV contamination of blood and blood products in the 1980s and 1990s, Ann. Intern. Med. (2002) 136(4): 312-9).

For another, small proteinaceous particles, such as prions, could be present and current scientific evidence suggests that even a very small number could result in disease. In a recent case, a healthy subject died from possible transmission of variant Creutzfeldt-Jakob disease from blood transfusion (Liewelyn et al., Possible transmission of variant Creutzfeldt-Jakob disease by blood transfusion, Lancet (2004) 363(9407): 417-21). This raises concerns regarding the consistent safety of blood and blood-derived products (Bird, S.M., Recipients of blood or blood products "at vCJD risk," BMJ (2004) 328(7432): 118-9). Because of such safety concerns, regulators are encouraging replacing animal components with recombinant proteins in vaccine production and other applications of cell culture techniques (Egan, W.M., Bovinederived products used in the manufacture and formulation of vaccines: current policies and issues for the future (2004), available at http://www.fda.gov/cber/summaries/pda092004we.pdf; European Medicine Agency (EMA), CPMP position statement on Crutzfeldt-Jakob disease and plasma-derived and urine-derived medicinal products (2003), available at http:l/www.emea.eu.intlpdfs/human/presslpos/287902en.pdf; World Health Organization (WHO), Variant Creutzfeldt-Jakob disease (2002), available at http://www.who.int/mediacentre/factsheets/fs180/en/). Further, many users may be hesitant to use products from cell culture media that contain animal components for religious or moral reasons (Sarkar, S., Use of animal products in vegetarians and others, Anaesthesia (2005) 60(5): 519-20).

Therefore, there is a need to produce components for incorporation into cell culture media that is not derived from, or produced by, animal sources, yet still retain their functional value for cell cultures.

### SUMMARY OF THE INVENTION

Accordingly, it is an objective of the invention to provide cell culture media or media supplement containing components derived from plant cells, which when used in cell culture, results in the same or improved growth and productivity of cells as compared to the use of media containing animal-derived components.

One embodiment of the invention comprises a cell culture media with at least one plant-produced heterologous protein as a cell culture media component.

Another embodiment of the invention comprises a method for producing an enhanced cell culture media, comprising producing at least one heterologous protein in a plant cell and combining the at least one plant-produced heterologous protein with a cell culture media.

Another embodiment of the invention comprises a cell culture media produced by a process comprising producing at least one heterologous protein in a plant cell and combining at least one plant-produced heterologous protein with a cell culture media.

Another embodiment of the invention discloses that in a cell culture media having one or more proteinaceous cell culture media components, the improvement comprising at least one plant-produced heterologous protein as a cell culture media component, wherein the heterologous protein is produced by the following process:
a) transforming a plant cell with a chimeric gene comprising:
   (i) a promoter from the gene of a seed storage protein;
   (ii) a first DNA sequence, operably linked to the promoter, encoding a seed storage protein, and
   (iii) a second DNA sequence, operably linked to the promoter, encoding the heterologous protein, wherein the first and second DNA sequences are linked in translation frame and together encode a fusion protein comprising the storage protein and the heterologous protein; and
b) growing a plant from the transformed plant cell for a time sufficient to produce seeds containing the heterologous protein. The embodiment may optionally include purifying the heterologous protein from the harvested seeds.

Another embodiment of the invention comprises a method for producing a cell culture media having at least one plant-produced heterologous protein as a cell culture media component, the method comprising:
a) transforming a plant cell with a chimeric gene comprising:
   (i) a promoter from the gene of a seed storage protein;
   (ii) a first DNA sequence, operably linked to the promoter, encoding a seed storage protein, and
   (iii) a second DNA sequence, operably linked to the promoter, encoding the heterologous protein, wherein the first and second DNA sequences are linked in translation frame and together encode a fusion protein comprising the storage protein and the heterologous protein;
b) growing a plant from the transformed plant cell for a time sufficient to produce seeds containing the heterologous protein;
c) harvesting the seeds from the plant; and
d) combining the heterologous protein with a cell culture media.

Another aspect of the invention comprises a method for creating a recombinant plant cell that can produce components that may be incorporated into cell culture media or supplement for cell culture media.

Another aspect of the invention comprises a method of transforming a plant cell by incorporating a DNA segment that encodes for a component of cell culture media.

Another aspect of the invention comprises a method of transforming a rice cell by incorporating a DNA segment that encodes for components of cell culture media such as amino acids, growth factors, and other cell culture proteins including lactoferrin and human serum albumin.

Another aspect of the invention comprises a cell culture medium with components that are derived from plants, and more preferably all the organic ingredients are derived exclusively from non-animal sources.

Another aspect of the invention comprises a method for the production of cell culture media, the method comprising incorporating into cell culture media components derived from recombinant plant cells.

Another aspect of the invention comprises of the use of the media supplemented with plant-derived components to improve cell growth and productivity in cell cultures.

Another aspect of the invention comprises improved cell culture media comprising one or more media ingredients and as a supplement to one or more cell culture media components derived from plant cells.

Another aspect of the invention comprises a method for achieving high growth rate of culture cells and high productivity of culture cells by culturing the culture cell in the improved cell culture media.

Another aspect of the invention comprises a method of producing an improved cell culture media comprising a) obtaining a plant cell transformed with a vector containing a plant-based promoter and a gene encoding a cell culture media component, b) cultivating the transformed plant cells to set seeds, c) harvesting the mature seeds, d) extracting and purifying of the cell culture components from the seed, and e) adding the components to cell culture media.

### BRIEF DESCRIPTION OF THE FIGURES

Figure 1 is a comparison of the codon-optimized epidermal growth factor sequence ("Egfactor") with a native epidermal growth factor sequence ("Native Gene"), aligned to show 53 codons in the mature sequences, with 27 (51 %) codon changes and 30 (19%) nucleotide changes.

Figure 2 is a restriction map of the 3,877 bp plasmid, API303 (pGt1-EGF v2.1), showing an expression cassette for epidermal growth factor (EGF), and containing a rice Gt1 promoter, a Gt1 signal peptide, codon optimized EGF, a Nos terminator and an ampicillin resistance selectable marker.

Figure 3 is a restriction map of the 4,142 bp plasmid, API270 (pGlb-EGF v2.1), showing an expression cassette for epidermal growth factor ("EGF"), and containing a Glb promoter, a Glb signal peptide, codon optimized EGF, a Nos terminator and an ampicillin resistance selectable marker.

Figure 4 is a Western blot analysis of recombinant human EGF ("rhEGF") in the R1 generation of transgenic rice seeds. Lane 1 indicates extracts from seeds of control untransformed Taipei 309 rice variety (Oryza sativa, Japonica). Lanes 2 to 5 show rhEGF expressed in the seed extracts obtained from independent transgenic rice events. Lane 6 shows a purified rhEGF standard expressed in yeast, loaded at 125 ng. Lane 7 shows a broad range of molecular weight markers.

Figure 5 is a comparison of the codon-optimized insulin-like growth factor I sequence ("Insgfact") with a native human insulin-like growth factor I sequence ("native gene"), aligned to show 70 codons in the mature sequences, with 40 (57%) codon changes and 47 (22%) nucleotides changes.

Figure 6 is a restriction map of the 4,194 bp plasmid, API271 (pGlb-IGF v2.1), showing an expression cassette for insulin-like growth factor I ("IGF"), and containing a Glb promoter, a Glb signal peptide, codon optimized IGF, a Nos terminator and an ampicillin resistance selectable marker.

Figure 7 is a restriction map of the 3,928 bp plasmid, API304 (pGt1-IFG v2.1), showing an expression cassette for insulin-like growth factor I ("IGF"), and containing a rice Gt1 promoter, a Gt1 signal peptide, codon optimized IGF, a Nos terminator and an ampicillin resistance selectable marker.

Figure 8 is a Western blot analysis of recombinant human IGF-I ("rhlGF") expressed in the R1 generation of transgenic rice seeds. Lane 1 shows rice seed extract from seeds of control untransformed rice variety Taipei 309. Lanes 2 to 8 show rhlGF expressed in seed extracts obtained from seven independent transgenic rice events. Lane 9 shows a purified rhlGF-1 standard expressed in yeast, loaded at 1 µg. Lane 10 shows a broad range of molecular weight markers.

Figure 9 is a comparison of the codon-optimized intestinal trefoil factor sequence ("Trefoil") with a native intestinal trefoil factor sequence ("Native Gene"), aligned to show 60 codons in the mature sequences, with 26 (43%) codon changes and 28 (15%) nucleotide changes.

Figure 10 is a restriction map of the 4,163 bp plasmid, API269 (pGlb-ITF-nos), showing an expression cassette for intestinal trefoil factor ("ITF"), and containing a Glb promoter, a Glb signal peptide, codon optimized ITF, a Nos terminator and an ampicillin resistance selectable marker.

Figure 11 is a restriction map of the 3,889 bp plasmid, API307 (pGt1-ITF-nos), showing an expression cassette for intestinal trefoil factor (ITF), and containing a rice Gt1 promoter, a Gt1 signal peptide, codon optimized ITF, a Nos terminator and an ampicillin resistance selectable marker.

Figure 12 is a Western blot analysis of recombinant human ITF ("rhITF") expression in the R1 generation of transgenic rice seeds. Lane 1 indicates extracts from seeds of control untransformed Taipei 309 rice variety. Lanes 2 and 3 show rhlTF expressed in the seed extracts obtained from two independent transgenic rice events. Lane 4 indicates a purified rhlTF standard expressed in yeast, loaded at 1 µg. Lane 5 shows a broad range of molecular weight markers.

Figure 13 is a comparison of the codon-optimized human lactoferrin sequence ("cod opt lac") with a native human lactoferrin sequence ("native lac"), aligned to show 693 codons, with 413 (59.6%) codon changes and 467 (22.5%) nucleotide changes.

Figure 14 is a plasmid map of the 5,817 bp plasmid, API164 (GT1-Lac), showing an expression cassette for human lactoferrin and containing a Gt1 promoter, a Gt1 signal peptide, codon optimized human lactoferrin, a Nos terminator and a kanamycin resistance selectable marker.

Figure 15 shows the results of a SDS-PAGE analysis for the expression of recombinant human lactoferrin. Total proteins from rice seed extracts were suspended in Laemli sample buffer, run on a gradient gel and stained with Coomassie blue. Lane 1 is the molecular weight marker. Lanes 3 to 6 are purified human derived lactoferrin (Sigma Chemical, USA). Lanes 8 to 13 are single seed extracts from homozygous independent transgenic rice lines and lane 14 is a seed extract from non-transformed rice variety Taipei 309.

Figure 16 is a stable expression of recombinant human lactoferrin in transgenic rice grains from R₂ through R₁₀ generations. Total soluble proteins from 1 g of brown rice flour was extracted with 40 ml of extraction buffer and clarified by centrifugation. The extract was analyzed via ELISA. Extraction was repeated three times and standard deviation is shown as an error bar.

Figure 17 shows DNA and protein sequence of fusion between Gt1 signal peptide (Gt1 SP) and codon-optimized human serum albumin (OPTIMIZED HSA) sequence based on native protein sequence derived from P02768 (Swiss-Prot).

Figure 18 is a plasmid map of the 5,496 bp plasmid, API504 (GT1-HSA), showing an expression cassette for human serum albumin and containing a Gt1 promoter, a Gt1 signal peptide, codon optimized human serum albumin, a Nos terminator and a kanamycin resistance selectable marker.

Figure 19 is a plasmid map of the 12,388 bp plasmid, API508 (JH/GT1-HSA), showing an expression cassette for human serum albumin and containing a Gt1 promoter, a Gt1 signal peptide, codon optimized human serum albumin, a Nos terminator and a kanamycin resistance selectable marker.

Figure 20 shows the effect of three forms of recombinant human lactoferrin on cell growth. The three forms of lactoferrin are asis-lactoferrin, apo-lactoferrin and holo-lactoferrin. Asis-lactoferrin is the same as partial-lactoferrin with approximately 50% iron saturation. The baseline is with basal media and the rest is baseline supplemented with various concentration of recombinant human lactoferrin as indicated in the graph. Cell growth is measured as thymidine incorporation.

Figure 21 shows the effect of three forms of recombinant human lactoferrin on cell growth. The three forms of lactoferrin are asis-lactoferrin, apo-lactoferrin and holo-lactoferrin. Asis-lactoferrin is the same as partial-lactoferrin with approximate 50% iron saturation. The baseline is with basal media plus 5% fetal calf serum and the rest are baseline supplemented with epidermal growth factor, 5% FCS or recombinant human lactoferrin at a final concentration of 1 mg/ml. Cell growth is measured as number of cells/ml.

Figure 22 shows cell culture media supplemented with recombinant human lactoferrin (LACROMIN^{™}) expressed in rice grain promotes hybridoma cell growth and increases hybridoma cell number as compared to animal derived transferrin. FBS is fetal bovine serum. AFM6 is serum-free cell culture media derived from KC Bio Kansas. AFM6-Fe is a serum-free media without iron.

Figure 23 shows the effect of recombinant human serum albumin (rHSA) and plasma-derived human serum albumin (pHSA) on the growth of hybridoma AE1 cells in control medium (CM) containing reduced serum (1% FBS).

Figure 24 shows the effect of recombinant human serum albumin (rHSA) and plasma-derived human serum albumin (pHSA) on the growth of hybridoma AE1 cells in serum-free medium (SMF).

### DETAILED DESCRIPTION

Unless otherwise indicated, all terms used herein have the meanings given below or are generally consistent with same meaning that the terms have to those skilled in the art of the present invention. Practitioners are particularly directed to Sambrook et al. (1989) Molecular Cloning: A Laboratory Manual (Second Edition), Cold Spring Harbor Press, Plainview, N.Y., Ausubel FM et al. (1993) Current Protocols in Molecular Biology, John Wiley & Sons, New York, N.Y., and Gelvin and Schilperoot, eds. (1997) Plant Molecular Biology Manual, Kluwer Academic Publishers, The Netherlands for definitions and terms of the art.

General and specific techniques for producing proteins from plant cells may be obtained from the following patents and applications, each of which is incorporated herein in its entirety by reference: U.S. Pat. Appl. Pub. No. 2003/0172403 A1 ("Plant Transcription Factors and Enhanced Gene Expression"); U.S. Pat. No. 6,991,824 ("Expression of Human Milk Proteins in Transgenic Plants"); U.S. Pat. Appl. Pub. No. 2003/0221223 ("Human Blood Proteins Expressed in Monocot Seeds"); U.S. Pat. Appl. Pub. No. 2004-0078851 ("Production of Human Growth Factors in Monocot Seeds"); U.S. Pat. Appl. Pub. No. 2004/0063617 ("Method of Making an Anti-infective Composition for Treating Oral Infections"); and international application no. PCT/US2004/041083 ("High-level Expression of Fusion Polypeptides in Plant Seeds Utilizing Seed-Storage Proteins as Fusion Carriers"). Other general and specific techniques for producing proteins from plant cells may be obtained, for example, from the following references, each of which is incorporated herein in its entirety by reference: U.S. Patent No. 5,693,507, U.S. Patent No. 5,932,479, U.S. Patent No. 6,642,053, and 6,680,426 (each titled "Genetic Engineering of Plant Chloroplasts"); U.S. Pat. Appl. Pub. No. 2005/0066384 ("Site-Targeted Transformation Using Amplification Vectors"); U.S. Pat. Appl. Pub. No. 2005/0221323 ("Amplification Vectors Based on Trans-Splicing"); U.S. Pat. Appl. Pub. No. 2006/0026718 ("Method of Controlling Cellular Processes in Plants"); and U.S. Pat. Appl. Pub. No. 2006/0075524 (Method of Controlling A Cellular Process in a Multi-Cellular Organism"); Marillonnet et al., Systemic Agrobacterium tumefaciens-mediated transfection of viral replicons for efficient transient expression in plants, Nature Biotech. (2005) 23(6): 718-723.

The polynucleotides of the invention may be in the form of RNA or in the form of DNA, and include messenger RNA, synthetic RNA and DNA, cDNA, and genomic DNA. The DNA may be double-stranded or single-stranded, and if single-stranded may be the coding strand or the non-coding (anti-sense, complementary) strand.

The term "stably transformed" with reference to a plant cell means the plant cell has a non-native (heterologous) nucleic acid sequence integrated into its genome which is maintained through two or more generations.

By "host cell" is meant a cell containing a vector and supporting the replication and/or transcription and/or expression of the heterologous nucleic acid sequence. Preferably, according to the invention, the host cell is a plant cell. Other host cells may be used as secondary hosts, including bacterial, yeast, Insect, amphibian or mammalian cells, to move DNA to a desired plant host cell.

A "plant cell" refers to any cell derived from a plant, including undifferentiated tissue (e.g., callus) as well as plant seeds, pollen, propagules, embryos, suspension cultures, meristematic regions, leaves, roots, shoots, gametophytes, sporophytes and microspores.

The term "mature plant" refers to a fully differentiated plant.

The term "seed product" includes; but is not limited to, seed fractions such as de-hulled whole seed, flour (seed that has been de-hulled by milling and ground into a powder) a seed extract, preferably a protein extract (where the protein fraction of the flour has been separated from the carbohydrate fraction), malt (including malt extract or malt syrup) and/or a purified protein fraction derived from the transgenic grain.

The term "biological activity" refers to any biological activity typically attributed to that protein by those skilled in the art.

"Monocot seed components" refers to carbohydrate, protein, and lipid components extractable from monocot seeds, typically mature monocot seeds.

"Seed maturation" refers to the period starting with fertilization in which metabolizable reserves, e.g., sugars, oligosaccharides, starch, phenolics, amino acids, and proteins, are deposited, with and without vacuole targeting, to various tissues in the seed (grain), e.g., endosperm, testa, aleurone layer, and scutellar epithelium, leading to grain enlargement, grain filling, and ending with grain desiccation.

"Maturation-specific protein promoter" refers to a promoter exhibiting substantially up-regulated activity (greater than 25%) during seed maturation.

The term "growth factor" refers to proteins, or biologically active fragments thereof, including, without limitation, epidermal growth factor (EGF), keratinocyte growth factors (KGF) including KGF-1 and KGF-2, insulin-like growth factors (IGF) including IGF-I and IGF-II, intestinal trefoil factor (ITF), transforming growth factors (TGF) including TGF-α and -β1-3, granulocyte colony-stimulating factor (GCSF), nerve growth factor (NGF) including NGF-β, and fibroblast growth factor (FGF) including FGF-1-19 and -12β, and biologically active fragments of these proteins. The sequences of these and other growth factors are well-known to those of ordinary skill in the art.

"Heterologous DNA" refers to DNA which has been introduced into plant cells from another source, or which is from a plant source, including the same plant source, but which is under the control of a promoter that does not normally regulate expression of the heterologous DNA.

"Heterologous protein" is a protein.encoded by a heterologous DNA. The proteins include, but are not limit to, growth factor(s), lactoferrin, transferrin, albumin, insulin, and fractions thereof, growth hormone and fractions thereof, Fibronectin (human) - attachment factor, Lamin (Mouse) - attachment factor, collagenase , platelet derived growth factor, Human brain-derived neurotrophic factor (BDNF), glial-derived neurotrophic factor (GDNF), thymic factors, haptocorin, lactahedrin, lactoperoxidase. Alpha-fetoprotein, immunoglobin, alpha-lactalbumin.

As used herein, the terms "native" or "wild-type" relative to a given cell, protein, polypeptide, nucleic acid, trait or phenotype, refers to the form in which that is typically found in nature.

As used herein, the term "purifying" is used interchangeably with the term "isolating" and generally refers to any separation of a particular component from other components of the environment in which it is found or produced. For example, purifying a recombinant protein from plant cells in which it was produced typically means subjecting transgenic protein-containing plant material to separation techniques such as sedimentation, centrifugation, filtration, and chromatography. The results of any such purifying or isolating step(s) may still contain other components as long as the results enrich for the component of interest.

As used herein, the terms "transformed" or "transgenic" with reference to a host cell means the host cell contains a non-native or heterologous or introduced nucleic acid sequence that is absent from the native host cell. Further, "stably transformed" in the context of the present invention means that the introduced nucleic acid sequence is maintained through two or more generations of the host, which is preferably (but not necessarily) due to integration of the introduced sequence into the host genome.

"Cell culture media" refers to media for cell culture purpose which includes complete media, basal media or basal media supplemented with cell culture media component, cell culture media supplement, or media with various amounts of serum or chemically defined media.

"Cell culture media component" refers any heterologous proteins for use as a supplement to cell culture media.

"Cell culture media ingredient" includes cell culture media components, proteins, peptides, hormones, carbohydrates, amino acids, lipids, vitamins, antibiotics, organic and inorganic salts.

"Cell culture media supplement" refers to a combination of one or multiple cell culture media components with or without other ingredients for addition to cell culture media.

Most cells in culture grow best at 37°C and at pH 7.4 with appropriate media. Typically, cell culture media contains carbohydrates, amino acids, various salts, bicarbonate to maintain pH, vitamins and hormones, and phenol red as pH indicator. Examples of cell culture media that can be formed include: Dulbecco's Modified Eagle's Medium (DME), Ham's Nutrient Mixtures, MCDB Media, Minimum Essential Medium Eagle, RPMI Media, Ames' Media, BGJb Medium (Fitton-Jackson Modification), Click's Medium, CMRL-1066 Medium, Fischer's Medium, Glascow Minimum Essential Medium (GMEM), Iscove's Modified Dulbecco's Medium (IMDM), L-15 Medium (Leibovitz), McCoy's 5A Modified Medium, NCTC Medium, Swim's S-77 Medium, Waymouth Medium, and William's Medium E.

Frequently, cell culture media is supplemented with serum at concentration of 5 to 10%. Serum is a complex mixture containing undefined essential matter for cell growth. Commonly used serum is fetal calf serum (FCS) and fetal bovine serum (FBS). Due to its nature of complex mixture, lack of consistency and potential risk of pathogen contamination, cell culture industries and regulatory agencies are seeking alternatives to adding serum to cell culture media.

Since cells grow poorly in basal media, the basal media is then supplemented with various growth factors, hormones, and other essential components to generate serum-free media but maintaining the same cell growth rate as in serum-based media. Human transferrin or bovine transferrin is added as a source of iron. Typically, serum-free media contains insulin, serum albumin, transferrin, insulin-like growth factor and epidermal growth factor. Other proteins such as lactoferrin have also been shown to be able to promote cell growth. Each component provides a distinct function in cell growth. In the art, these protein components are animal-derived. With the increase in the concern of potential pathogen contamination from animal sourced material, cell culture industries are trying to develop animal-component-free media. While some successes have been made with the use of plant-based hydrolysates or recombinant protein produced with microbial system, plant-based hydrolysate is an undefined mixture of components and recombinant protein from microbial sources are too expensive to use in routine cell culture. An alternative source of non-animal-derived components is urgently needed for cell culture industries.

The cell culture media components produced by the methods to be described in the following sections are entirely plant-based and may be combined with inorganic salts such as NaCl, KCl, NaH₂PO₄, NaHCO₃ CaCl₂, and MgCl₂ and other ingredients such as amino acids, vitamins, growth factors, sugars, and antibiotics to form a variety of different cell culture media.

Expression vectors for use in the present invention are chimeric nucleic acid constructs (or expression vectors or cassettes), designed for operation in plants, with associated upstream and downstream sequences.

In general, expression vectors for use in practicing the invention include the following operably linked elements that constitute a chimeric gene: a promoter derived from a gene encoding a plant protein, operatively linked to a gene encoding a heterologous protein. The vector can include a promoter from the gene of a maturation-specific monocot plant storage protein, a first DNA sequence, operably linked to the promoter, encoding a monocot plant seed-specific signal sequence (such as an N-terminal leader sequence or a C-terminal trailer sequence) capable of targeting a polypeptide linked thereto to an endosperm cell, preferably an endosperm-cell organelle, such as a protein storage body, and a second DNA sequence, linked in translation frame with the first DNA sequence, encoding a cell culture media component. The signal sequence is preferably cleaved from the cell culture media component in the plant cell.

The chimeric gene, in turn, is typically placed in a suitable planttransformation vector having (i) companion sequences upstream and/or downstream of the chimeric gene which are of plasmid or viral origin and provide necessary characteristics to the vector to permit the vector to move DNA from bacteria to the desired plant host; (ii) a selectable marker sequence; and (iii) a transcriptional termination region generally at the opposite end of the vector from the transcription initiation regulatory region.

Numerous types of appropriate expression vectors, and suitable regulatory sequences are known In the art for a variety of plant host cells. The promoter region is chosen to be regulated in a manner allowing for induction under seedmaturation conditions. In one aspect of this embodiment of the invention, the expression construct includes a promoter which exhibits specifically up-regulated activity during seed maturation. Promoters for use in the invention are typically derived from cereals such as rice, barley, wheat, oat, rye, corn, millet, triticale or sorghum. Examples of such promoters include the maturation-specific promoter region associated with one of the following maturation-specific monocot plant storage proteins: rice glutelins, oryzins, and prolamines, barley hordeins, wheat gliadins and glutelins, maize zeins and glutelins, oat glutelins, and sorghum kafirins, millet pennisetins, and rye secalins.

One embodiment of the present invention involves high-level expression of heterologous molecules that are components of cell culture media. These components can be expressed in a plant cell by a chimeric gene comprising the gene(s) encoding the desired component operably linked with an endogenous plant protein encoding gene. In a preferred embodiment the endogenous plant encoding gene is a rice seed storage protein gene for expression in a rice mature seed expression system.

The expressed polypeptide may be a multi-domain polypeptide with one domain being the heterologous or exogenous cell culture component and the other being the endogenous plant protein. At least one selective purification tag and/or at least one specific protease cleavage site may be provided for eventual release of the cell culture media component from the monocot seed storage protein carrier. For example, a strategic methionine or tryptophan residue providing a chemical cleavage site may be engineered in frame between the domains for release of the cell culture component from the endogenous plant protein. This technique is useful for cell culture media components that have protein portions or are proteins, for example lactoferrin, human serum albumin, and human growth factors.

Other selective protease cleavage sites include; but are not limited to enterokinase (ek), Factor Xa, thrombin, V8 protease, GENENASE™(a variant of subtilisin BPN'), α-lytic protease or tobacco etch virus protease. Alternatively, cleavage of the fusion protein could be performed via chemical cleaving agents such as cyanogen bromide or N-chlorosuccinimide.

The invention provides cell culture media components recombinantly produced in host monocot plant seed wherein the cell culture media component expressed comprises about 3% or greater of the total soluble protein in the monocot seed. Thus, for example, the yield of total soluble protein which comprises the cell culture media component targeted for production can be from about 3% to about 5%, from about 5% to about 10%, from about 10% to about 15%, from about 15% to about 20%, from about 20% to about 25%, and from about 25% to about 30% of the total soluble protein found in the recombinantly engineered production plant seed. Additionally, for example, the yield of total soluble protein which comprises the cell culture media component targeted for production can be about 3% or greater, about 5% or greater, about 10% or greater, about 15% or greater, about 20% or greater, and about 25% or greater.

An embodiment of the present invention is a cell culture media, comprising at least one plant-produced heterologous protein as a cell culture media component. In some embodiments, the plant is a monocot plant, such as rice, barley, wheat, rye, corn, millet, triticale, or sorghum, preferably rice. The heterologous protein is a plant protein or a non-plant protein, preferably human, which may be selected in some embodiments from the group consisting of growth factors, lactoferrin, transferrin, serum albumin, insulin, growth hormone, and fractions thereof, fibronectin attachment factor, lamin attachment factor, collagenase, platelet derived growth factor, brain-derived neutrophic factor, glial-derived neurotrophic factor, thymic factors, haptocorin, lactahedrin, lactoperoxidase, alpha-fetoprotein, immunoglobin, or alpha-lactalbumin. In some embodiments of the present invention, the growth factors are preferably epidermal growth factors, keratinocyte growth factors, insulin-like growth factors, intestinal trefoil factors, transforming growth factors, granulocyte colony-stimulating factors, nerve growth factors, fibroblast growth factors, or biologically active fragments thereof.

In some embodiments, the cell culture media is a reduced serum or serum-free medium. In other embodiments, the cell culture media is disclosed as a complete media, basal media, or basal media supplemented with a cell culture media component.

Another embodiment of the present invention is a method for producing a cell culture media, comprising producing at least one heterologous protein in a plant cell and combining the at least one plant-produced heterologous protein with a cell culture media.

Another embodiment of the present invention is a cell culture media produced by a process comprising producing at least one heterologous protein in a plant cell and combining at least one plant-produced heterologous protein with a cell culture media.

Another embodiment of the present invention is an improved cell culture media having one or more proteinaceous cell culture media components, the improvement comprising at least one plant-produced heterologous protein as a cell culture media component, wherein the heterologous protein is produced by the following process:
a) transforming a plant cell with a chimeric gene comprising:
   (i) a promoter from the gene of a seed storage protein;
   (ii) a first DNA sequence, operably linked to the promoter, encoding a seed storage protein, and
   (iii) a second DNA sequence, operably linked to the promoter, encoding the heterologous protein, wherein the first and second DNA sequences are linked in translation frame and together encode a fusion protein comprising the storage protein and the heterologous protein; and
b) growing a plant from the transformed plant cell for a time sufficient to produce seeds containing the heterologous protein. In some embodiments, the heterologous protein is purified from the harvested seeds. In some embodiments, the heterologous protein constitutes at least about 3.0% of the total soluble protein in the harvested seeds.

Another embodiment of the present invention is a method for producing a cell culture media having at least one plant-produced heterologous protein as a cell culture media component, the method comprising:
a) transforming a plant cell with a chimeric gene comprising:
   (i) a promoter from the gene of a seed storage protein;
   (ii) a first DNA sequence, operably linked to the promoter, encoding a seed storage protein, and
   (iii) a second DNA sequence, operably linked to the promoter, encoding the heterologous protein, wherein the first and second DNA sequences are linked in translation frame and together encode a fusion protein comprising the storage protein and the heterologous protein;
b) growing a plant from the transformed plant cell for a time sufficient to produce seeds containing the heterologous protein;
c) harvesting the seeds from the plant; and
d) combining the heterologous protein with a cell culture media.

Another embodiment of the present invention is a method of producing monocot seeds, preferably rice grains, that can express the desired cell culture media component, comprising:
a) transforming a monocot plant cell with a chimeric gene comprising:
   (i) a promoter from the gene of a monocot seed storage protein;
   (ii) a first DNA sequence, operably linked to the promoter, encoding a monocot seed storage protein; and
   (iii) a second DNA sequence, operably linked to the promoter, encoding a cell culture media component,
      wherein the first and second DNA sequences are linked in translation frame and together encode a fusion protein comprising the storage protein and the cell culture media component;
b) growing a monocot plant from the transformed monocot plant cell for a time sufficient to produce seeds containing the cell culture media component; and
c) harvesting the seeds from the monocot plant.

Another aspect of the present invention is a method of utilizing monocot seed storage proteins as fusion protein carriers, comprising the above method.

The invention also includes a chimeric gene, comprising:
(i) a promoter from the gene of a monocot seed storage protein;
(ii) a first DNA sequence, operably linked to the promoter, encoding a monocot seed storage protein; and
(iii) a second DNA sequence, operably linked to the promoter, encoding a cell culture media component;
wherein the first and second DNA sequences are linked in translation frame and together encode a fusion protein comprising the storage protein and the cell culture media component.

The monocot seed storage protein may be at the N-terminal or C-terminal side of the cell culture media component in the fusion protein. It is preferred that the monocot seed storage protein by located at the N-terminal side of the cell culture media component.

Preferably, the monocot plant comprises rice, barley, wheat, rye, corn, millet, triticale, or sorghum. More preferably, the monocot plant is rice.

All publications, patents and patent applications are herein expressly incorporated by reference in their entirety to the same extent as if each individual publication, patent or patent application was specifically and indicated individually to be incorporated by reference in its entirety.

The following examples illustrate but are not intended in any way to limit the scope of the invention.

### EXAMPLES

### Example 1: Expression of recombinant human growth factors

In general, expression vectors were constructed using standard molecular biological techniques. The vectors contain a heterologous protein coding sequence for certain growth factors under the control of a rice tissue-specific promoter, as further described below.

The nucleotide sequence of the promoter and the nucleotide sequence of the signal peptide of the rice glutelin-1 (Gt1) gene were cloned based on the published Gt1 gene sequence. The nucleotide sequence of the promoter and the nucleotide sequence of the signal peptide of the rice globulin (Glb) gene were cloned based on the published Gib gene sequence.

### A. Generation of human EGF expression vector

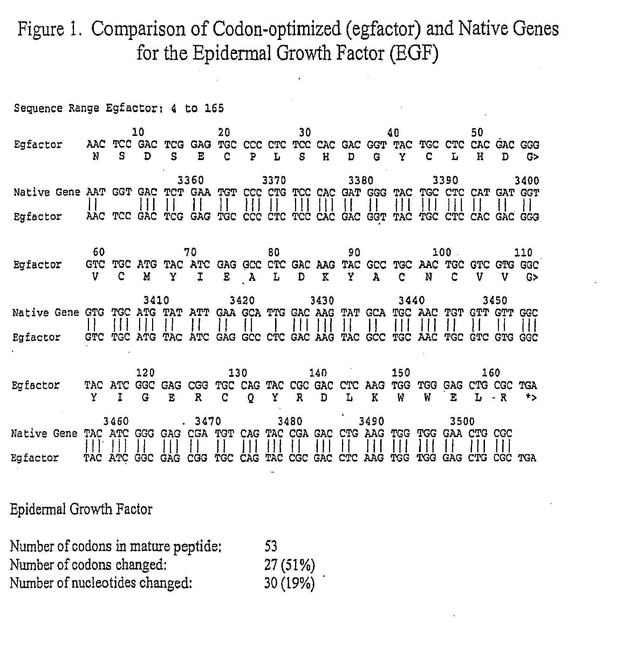

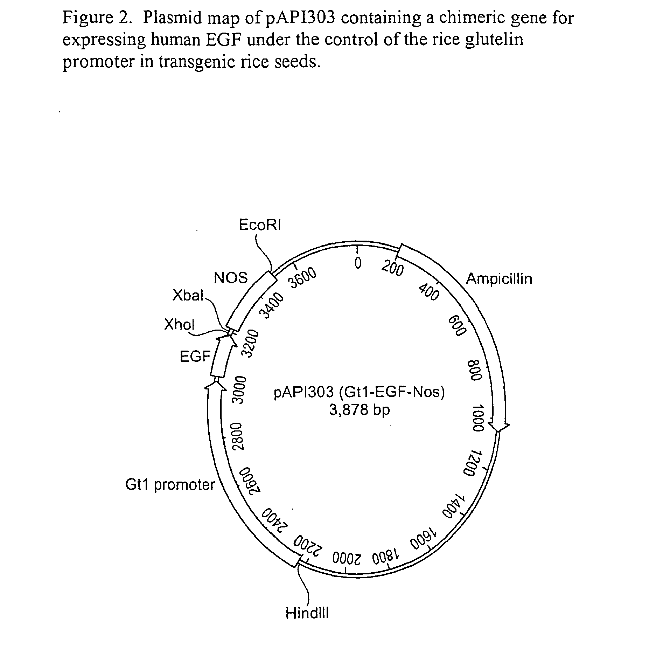

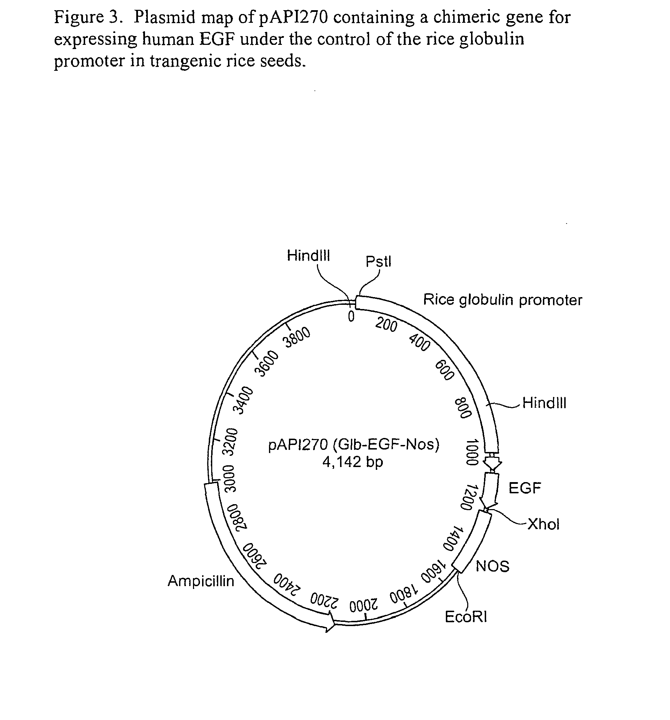

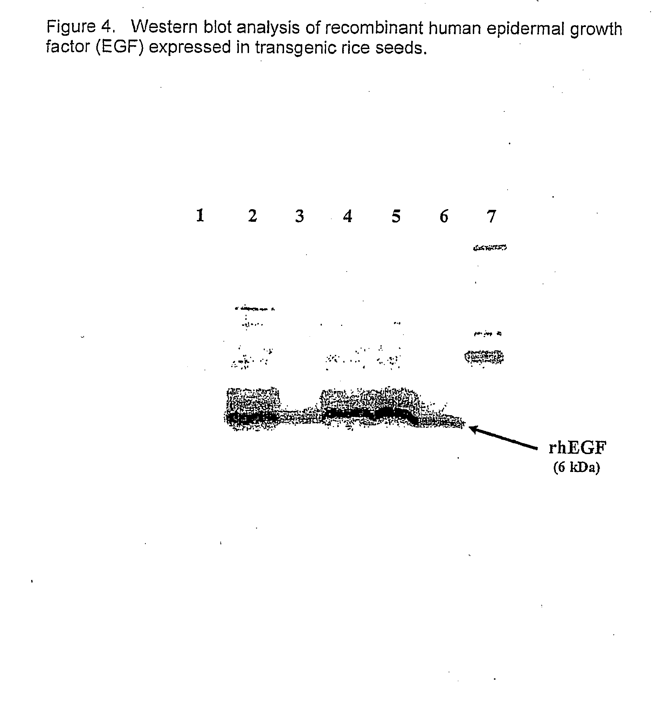

The human EGF gene was codon optimized as shown in Figure 1, and synthesized by Operon Technologies (CA, USA) (SEQ ID NO: 1). For expression of EGF in rice seeds, the codon optimized gene was operably linked to the rice endosperm specific glutelin (Gt1) promoter, Gt1 signal peptide and NOS terminator in pAPI303 (Figure 2), and to the rice endosperm specific globulin (Glb) promoter, Glb signal peptide and NOS terminator in pAPI270 (Figure 3). The transgenic plant expressing EGF was generated, and plant-generated recombinant EGF was detected, as shown in Figure 4 and as exemplified herein.

### B. Generation of human IGF-I expression vector

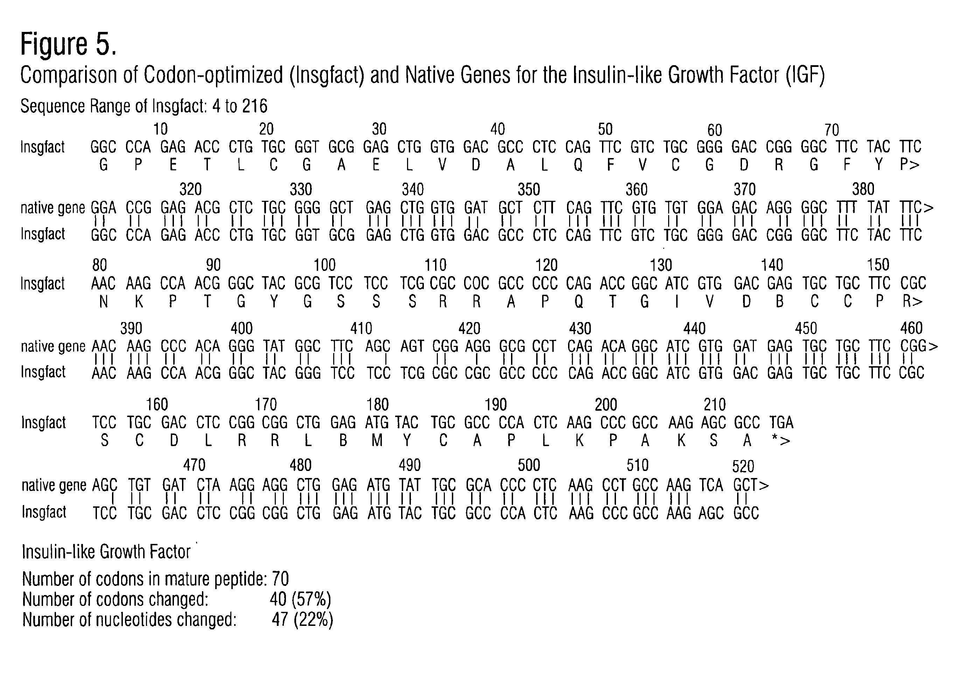

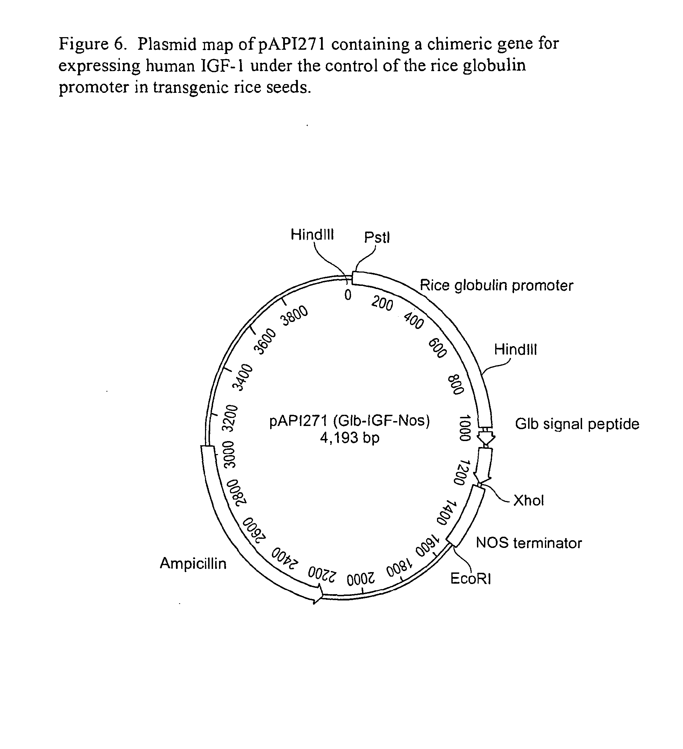

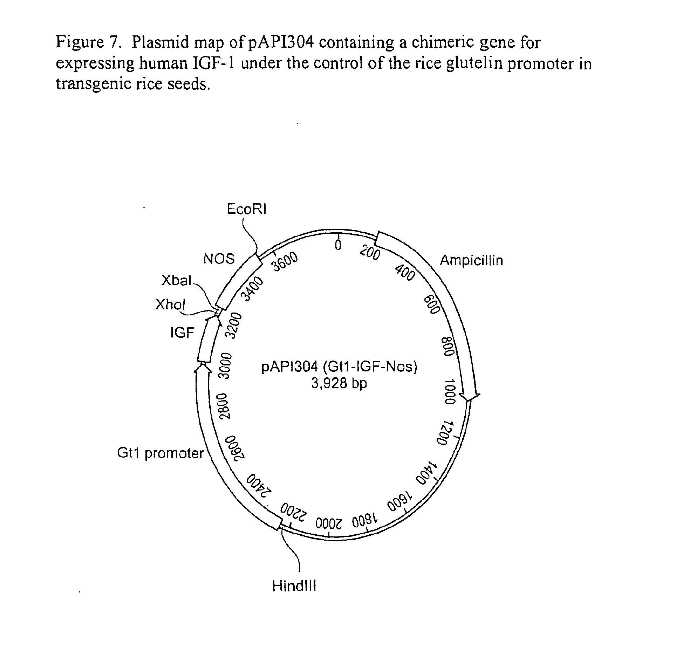

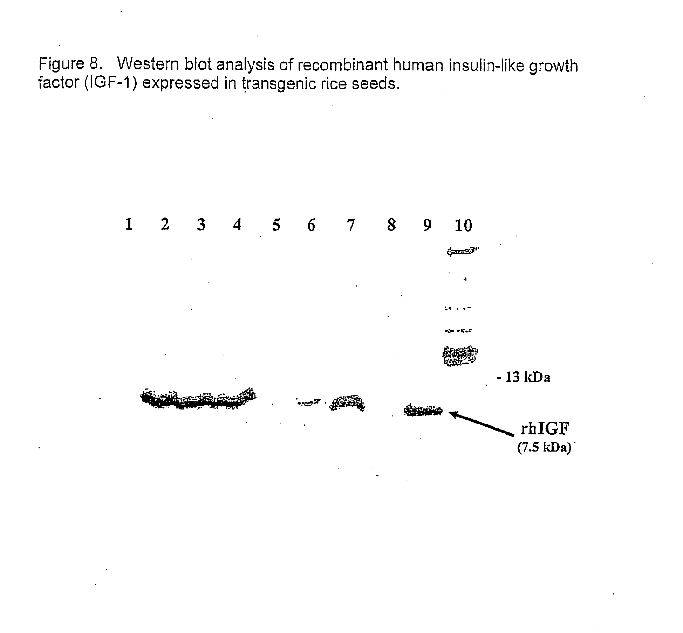

The IGF-I gene was codon optimized as shown in Figure 5, and synthesized by Operon Technologies (CA, USA) (SEQ ID NO: 3). For expression of IGF-I In rice seeds, the codon optimized gene was operably linked to the rice endosperm specific glutelin (Gt1) promoter, Gt1 signal peptide and NOS terminator in pAPI304 (Figure 7), and to the rice endosperm specific globulin (Glb) promoter, Glb signal peptide and NOS terminator in pAPI271 (Figure 6). The transgenic plant expressing IGF-I was regenerated, and plant-generated recombinant IGF-I was detected as shown in Figure 8 and as exemplified herein.

### C. Generation of human ITF

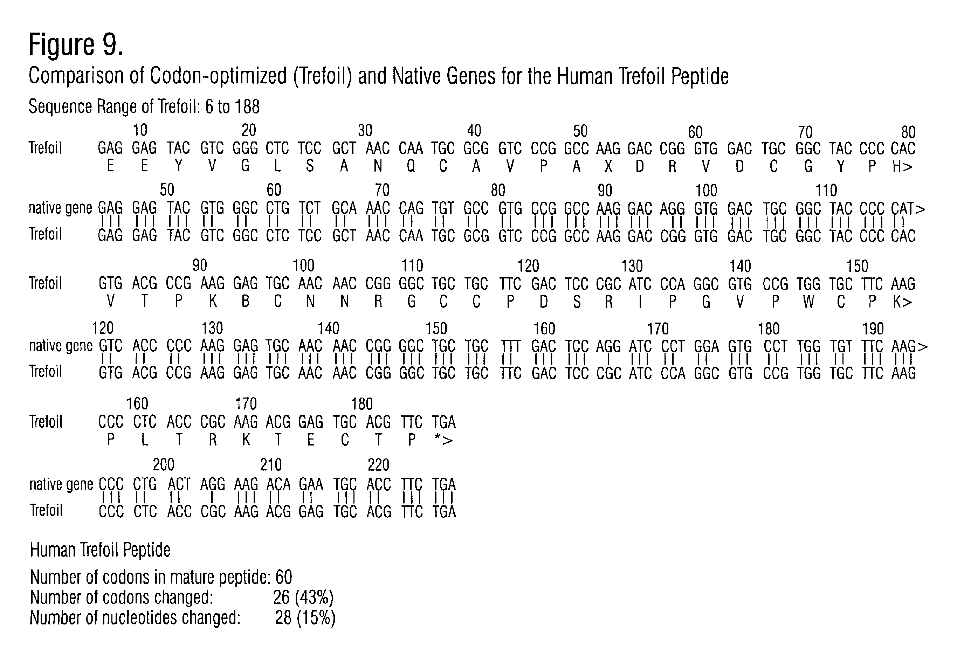

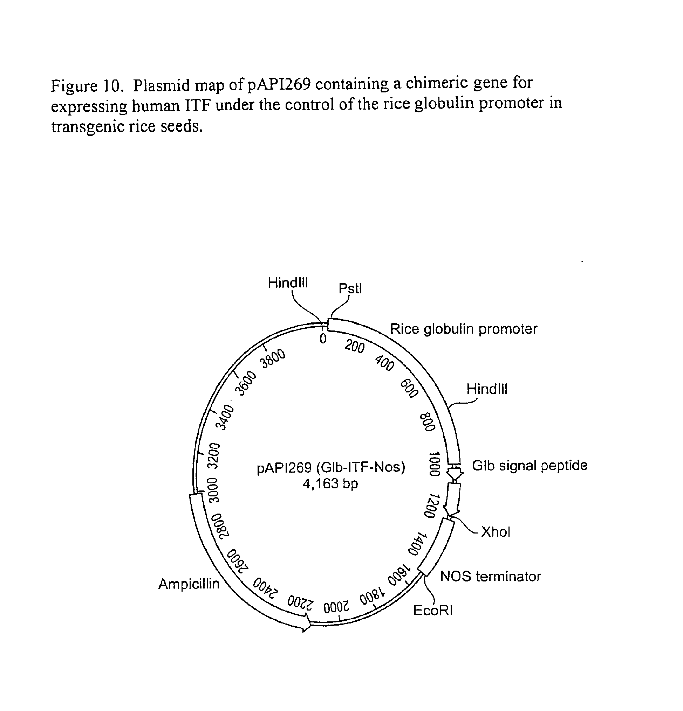

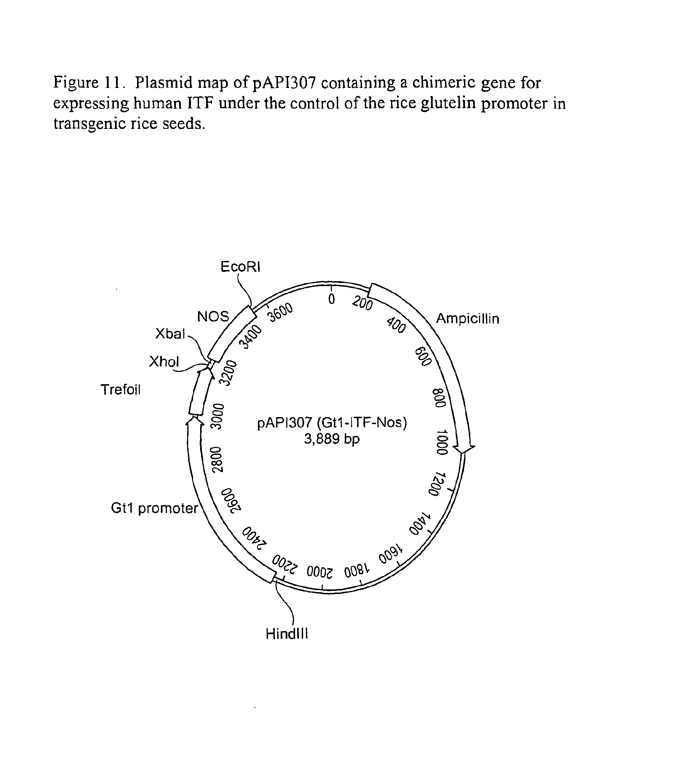

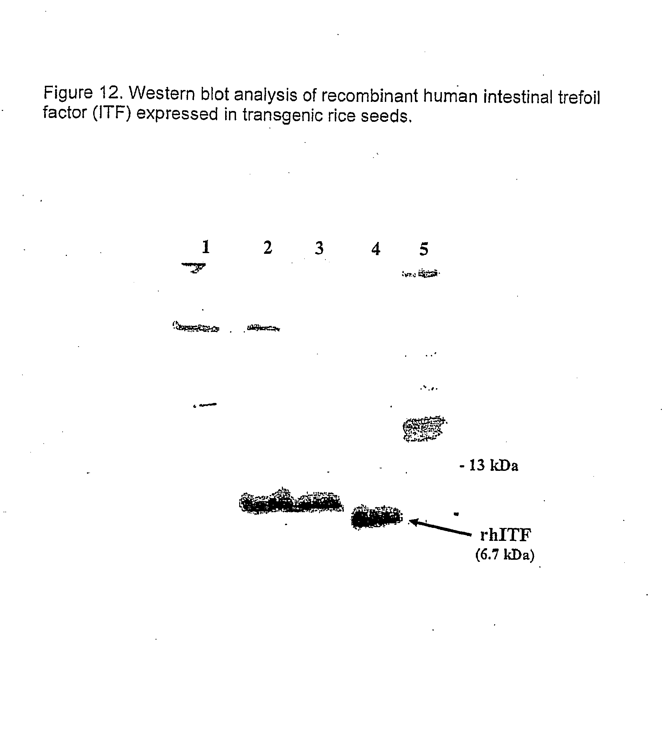

The ITF gene was codon optimized as shown in Figure 9, and synthesized by Operon Technologies (CA, USA) (SEQ ID NO: 5). For expression of ITF in rice seeds, the codon optimized gene was operably linked to the rice endosperm specific glutelin (Gt1) promoter, Gt1 signal peptide and NOS terminator in pAPI307 (Figure 11), and to the rice endosperm specific globulin (Glb) promoter, Glb signal peptide and NOS terminator in pAPI269 (Figure 10). The transgenic plant expressing ITF was generated, and plant-generated recombinant ITF was detected as shown in Figure 12 and as exemplified herein.

### D. Western blot analysis for all growth factors

Both untransformed (rice variety Taipei 309) and transgenic rice seeds (~10 pooled R1 seed from individual transgenic plants expressing either EGF, IGF-I or ITF) were ground in 1 ml of 0.35 M NaCl in phosphate buffered saline (PBS), pH 7.4, using an ice-cold mortar and pestle. The resulting extract was centrifuged at 14,000 rpm at 4°C for 10 min. Cleared supernatant was collected and -20 ug of this soluble protein extract was re-suspended in sample loading buffer, and loaded onto a pre-cast 10-20% polyacrylamide tricine gel (Novex) and subjected to SDS-PAGE. After electrophoresis, the gel was blotted to a 0.45 µm nitrocellulose membrane. The membrane was blocked with 5% non-fat dry milk in PBS pH 7.4 for 2 hrs followed by three washes with PBS for 10 min each. A primary rabbit polyclonal antibody prepared against EGF(Sigma), IGF-I (Sigma) or ITF (GI Company) was used at 1:2000 dilution in PBS. Bands were developed using goat anti-rabbit antibody coupled to the BCIP/NBT substrate system (Sigma). Results are shown in Figures 4, 8 and 12, respectively. Example 2: Expression of recombinant human lactoferrin

### A. An Expression Vector For Human Lactoferrin Expression In Transgenic Rice

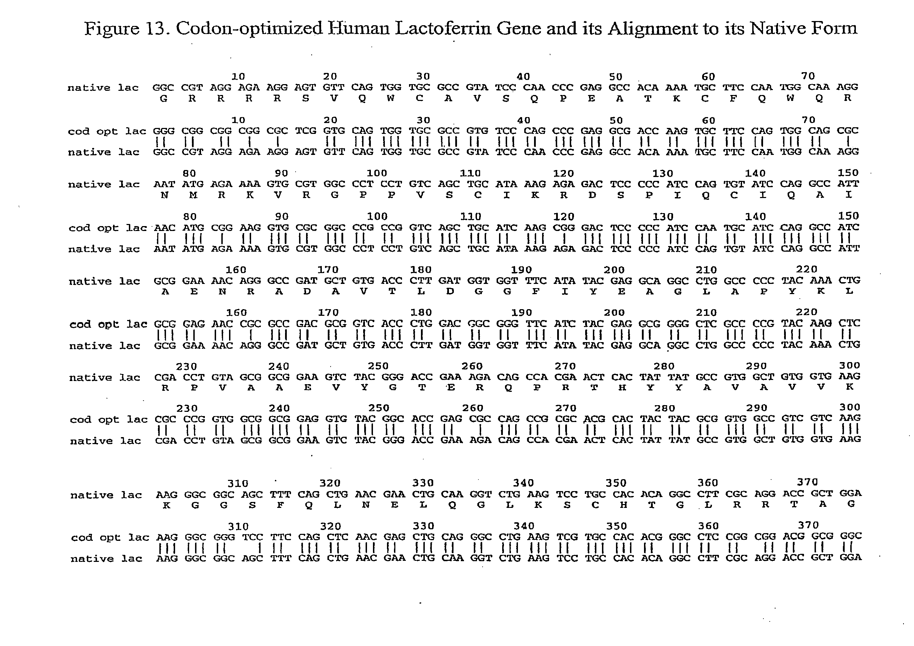

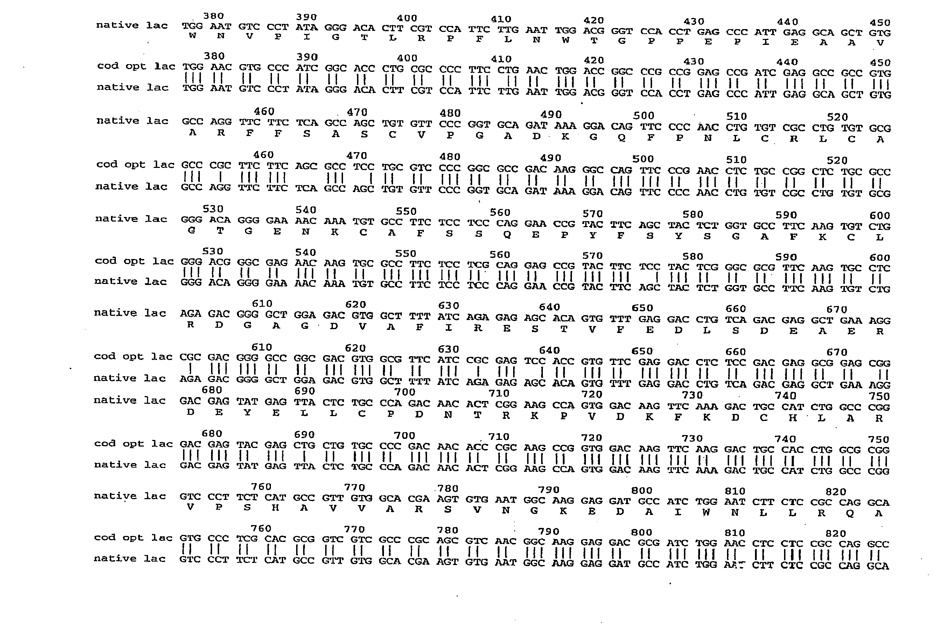

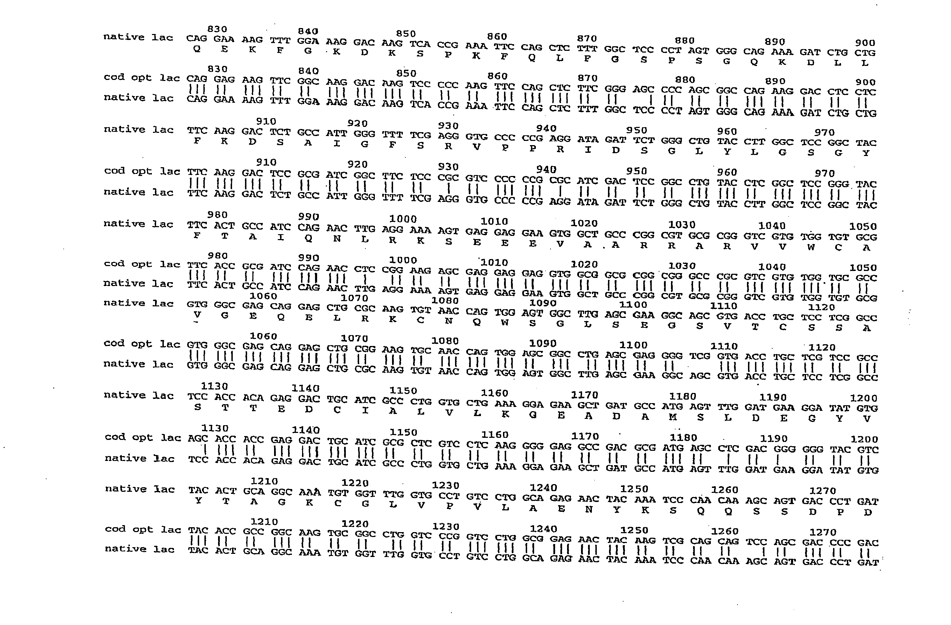

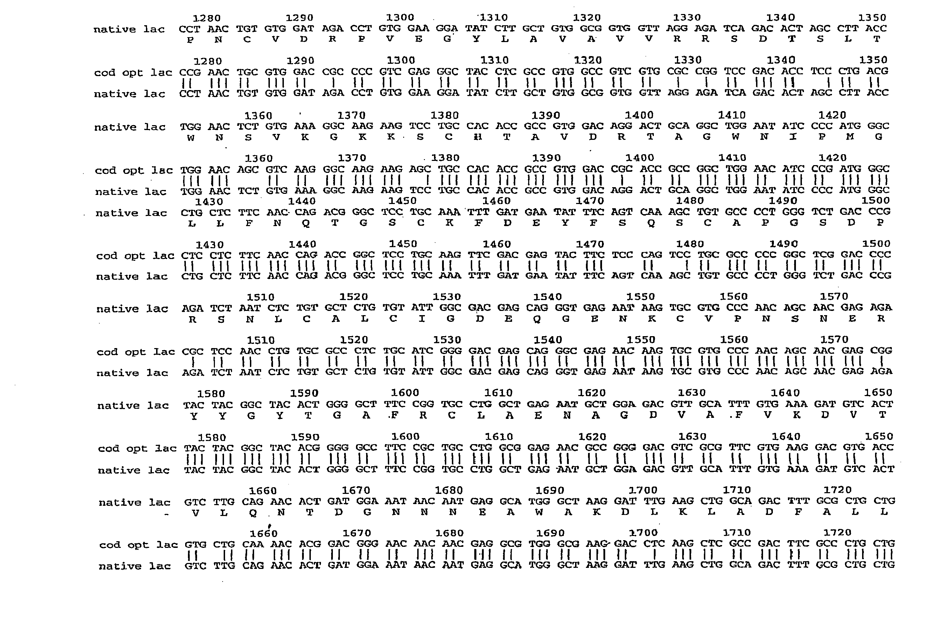

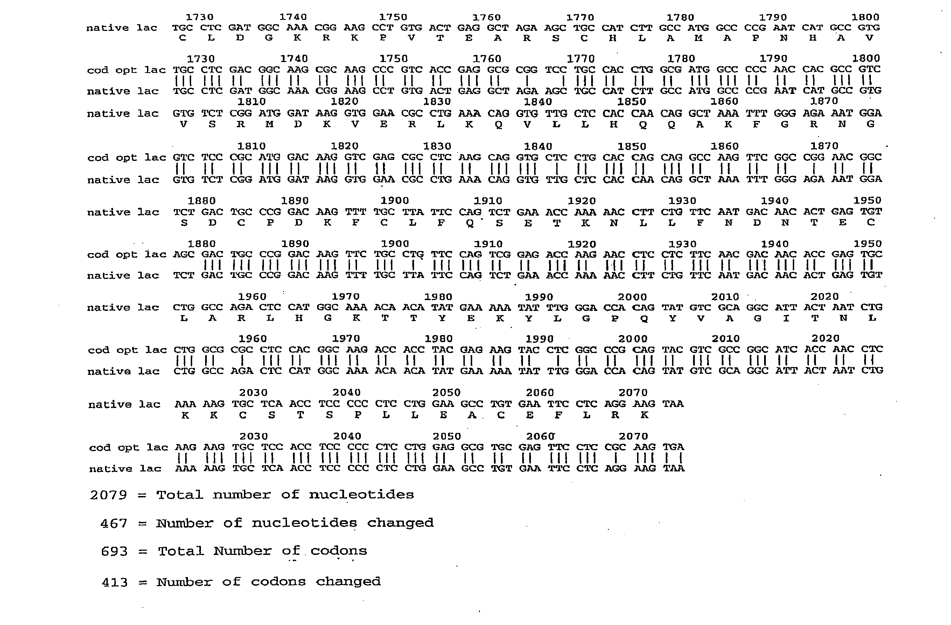

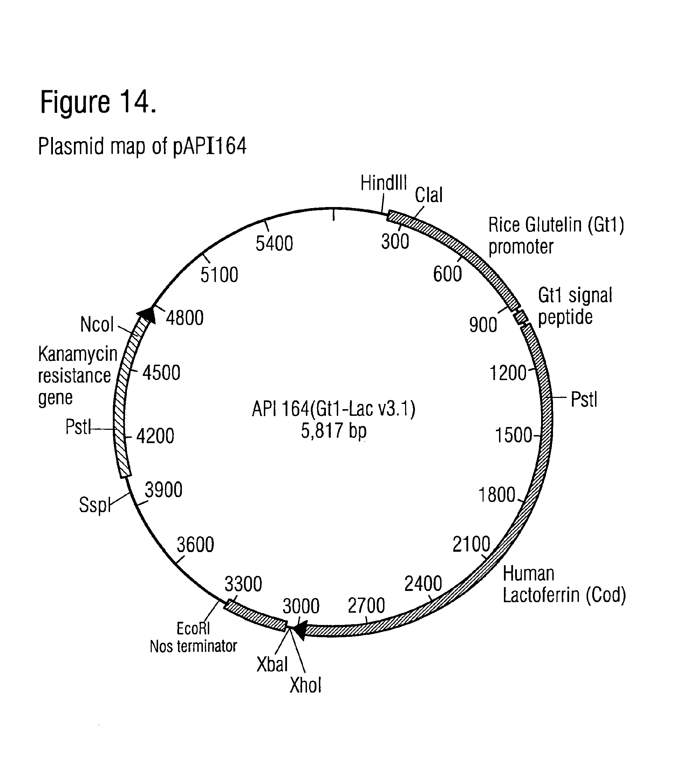

Complete nucleotide sequence of human mammary gland lactoferrin was codon optimized and synthesized by Operon Technologies (CA, USA). Human milk lactoferrin gene (Genbank accession number: HSU07642) was re-synthesized with codons most frequently used in translation of rice seed proteins in order to obtain optimal level of expression (Figure 13). Although numbers of codons changed accounted for 22.46% of the entire sequence, amino acid composition remains identical to native human lactoferrin. The plasmid containing the codon-optimized gene was called Lac-ger. Lac-ger was digested with SmaI/XhoI and the fragment containing the lactoferrin gene was cloned into pAPI141 that was partially digested with Nael and completely digested with XhoI. For expression of hLF in rice seeds, the codon-optimized gene was operably linked to the rice endosperm-specific glutelin (Gt1) promoter and NOS terminator. The resulting plasmid was designated pAPI164 (Figure 14).

### B. Production system

Rice variety Taipei 309 (Oryza sativa, Japonica) was selected as the production system for recombinant human lactoferrin (rhLF) and transgenic rice events were generated by the particle bombardment of embryogenlc rice calli with the plasmid pAPI164 and a companion marker plasmid containing the hygromycin phosphotransferase gene as a selectable marker. Fully developed, fertile rice plants were obtained by this procedure.

### C. High Level Protein Expression of recombinant human lactoferrin in rice grain

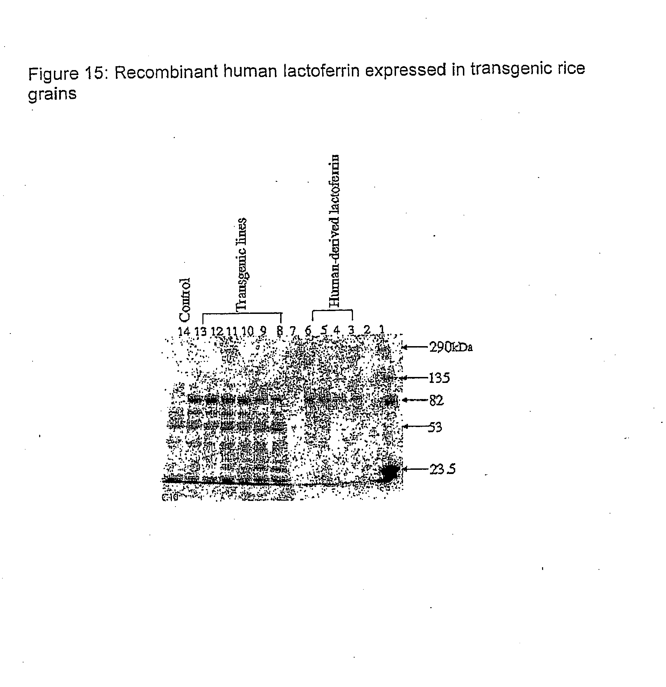

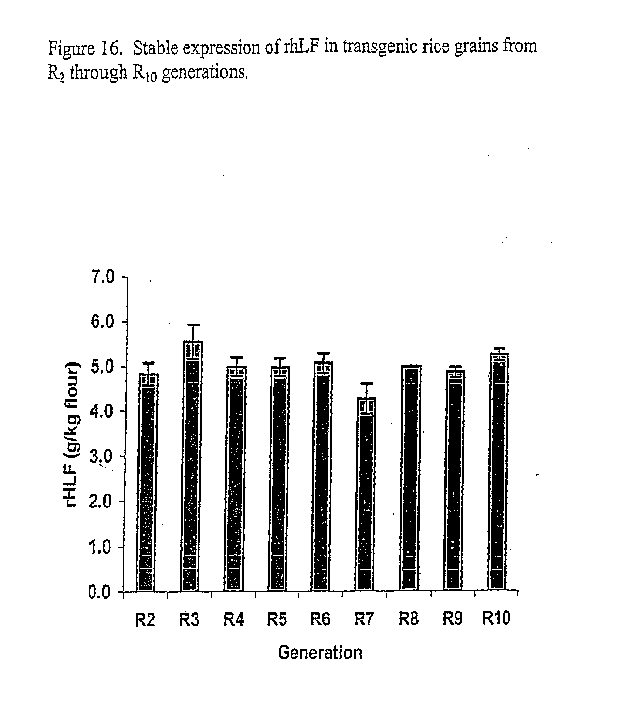

Expression of recombinant human lactoferrin was under the control of the seed maturation-specific promoter Gt1. The high level expression of recombinant human lactoferrin is evident in Figure 15 as independent transgenic rice events were screened. Total soluble proteins from mature rice seed extracts were run on Laemli gels and stained with Coomassie blue to visualize the proteins. An ~80kD recombinant lactoferrin protein was obtained in all transgenic lines as indicated by the stained gel. Expression levels of recombinant human lactoferrin corresponded to 0.5% of seed weight. The stable expression of recombinant human lactoferrin was monitored for 10 generations. The expression level is maintained at approximately 0.5% of seed weight of brown rice (Figure 16).

### Example 3: Expression of recombinant human serum albumin in rice grain

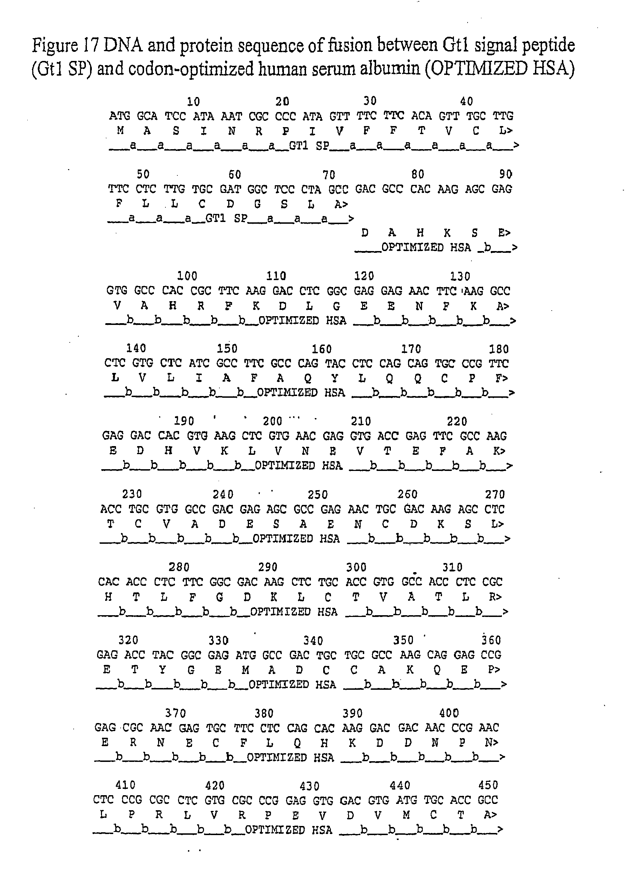

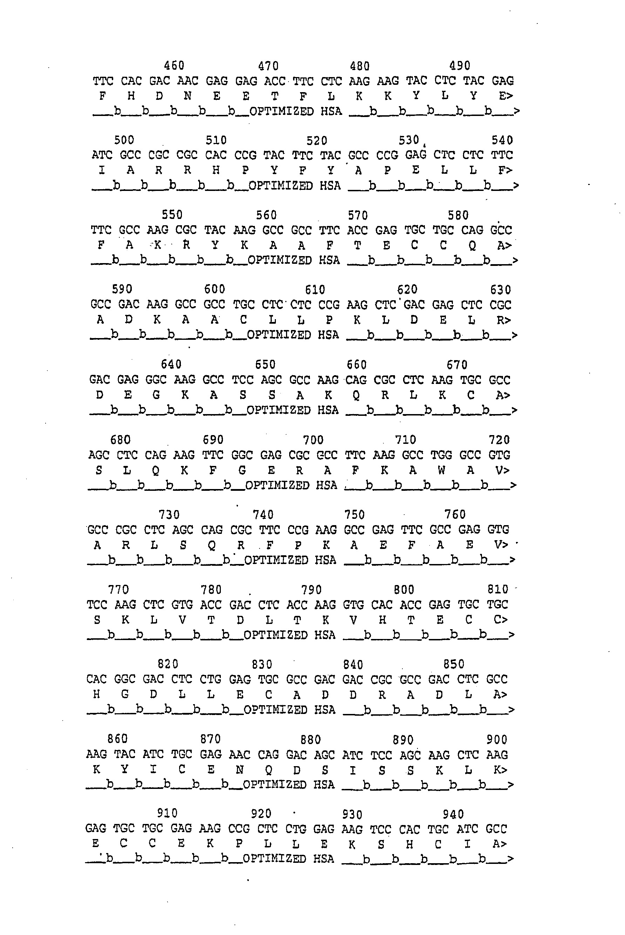

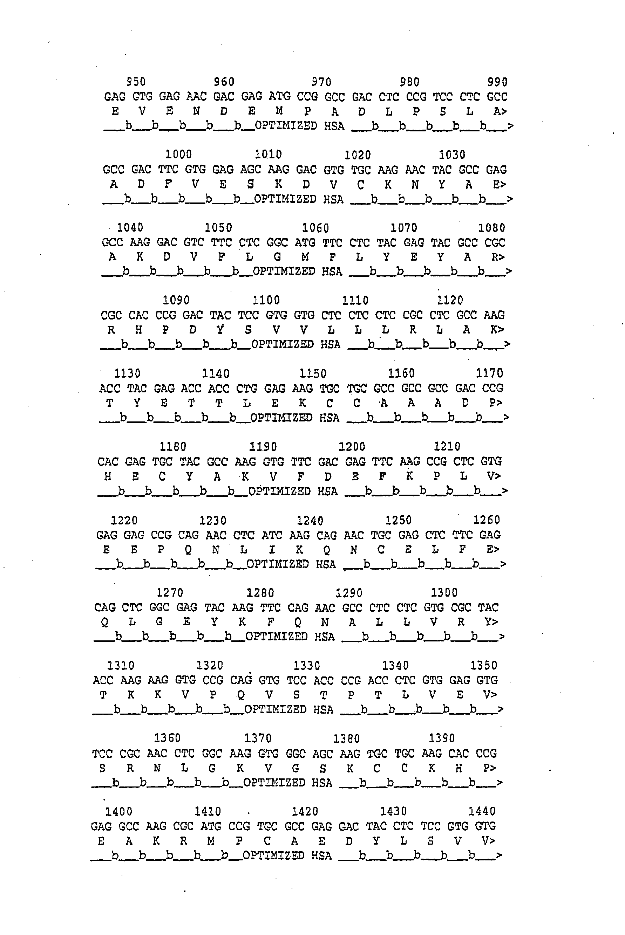

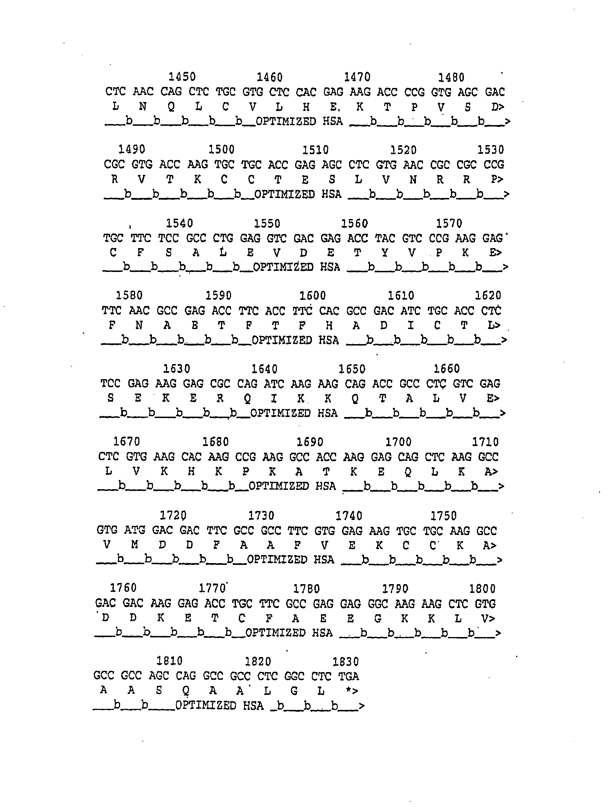

Protein sequences of human serum albumin (HSA) from various data bases were compared. The consensus sequence represented by accession number P02768 (Swiss-Prot) was used as a base for gene codon-optimization for suitable expression of human serum albumin in rice grain (Figure 17). Gene synthesis was carried out by Blue Heron (Seattle, WA) and the synthetic fragment was inserted into pUC based vector to create pUC-HSA. After confirmation of correct DNA and protein sequences, pUC-HSA was digested with Mlyl and Xhol. The fragment containing codon-optimized HSA gene was inserted into pAP1405, which had been precut with Nael and XhoI. Plasmid API405 was a derivative of pAPI141 which included Gt1 promoter, Gt1 signal sequence and a nos terminator.

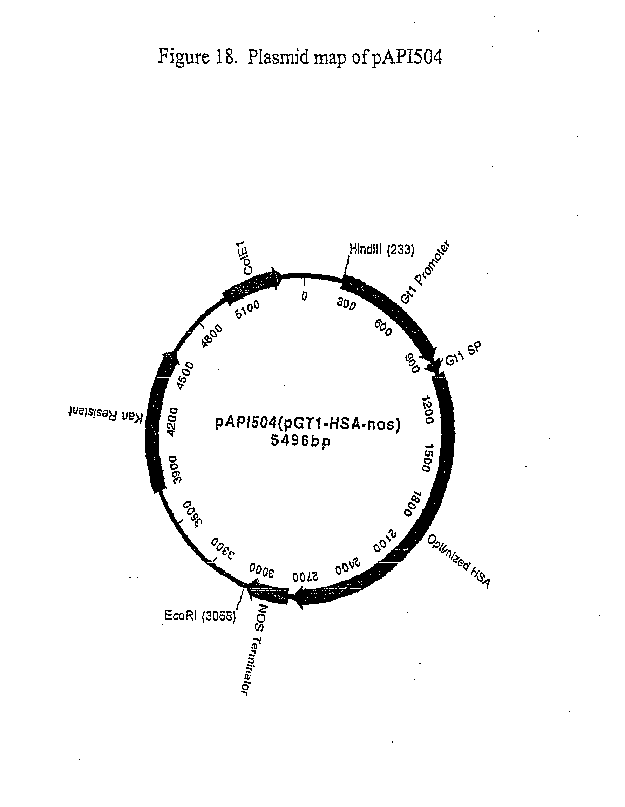

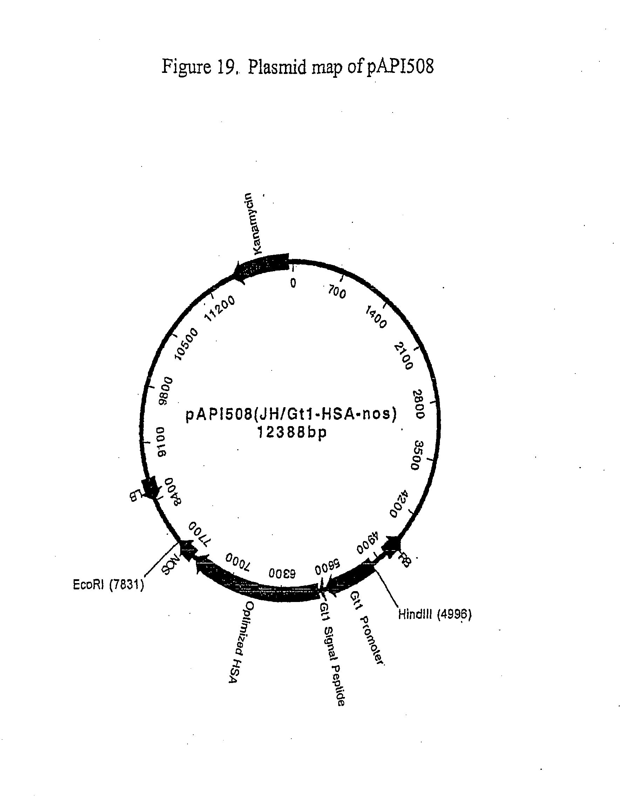

insertion of MlyI/XhoI fragment into pAPI405 resulted In pAPI504 (Figure 18). Plasmid API504 was then cleaved with HindIII and EcoRI. The HindIII/EcoRI fragment containing the entire expression cassette, Gt1 promoter, Gt1 signal sequence, codon-optimized HSA gene and nos terminator, was cloned into pJH2600 predigested with the same enzyme resulting in pAPI508 (Figure 19). Plasmid JH2600 was a shuttle vector between E coli and Agrobacterium. After DNA sequence verification, pAPI508 was moved into Agrobacterium AGL-1 for rice transformation. Plasmid API504 was also used via bombardment-based transformation following the procedure described previously. Upon transformation, transgenic plants were generated and were sent to greenhouse where transgenic R0 plants grew to maturity and set R1 seeds.

To monitor the expression of HSA in rice seeds, 10 R1 seeds from each R0 plant were extracted using 10 mL of extraction buffer (50 mM Tris-HCl pH 8.0. 150 mM NaCl). The supernatant was collected and the expression level from rice extracts was monitored by an ELISA (Bethyl Laboratories, Montgomery, TX). The results showed that the HSA expression level in rice transgenic seeds ranges from 0.01 to 0.85% of brown flour weight (0.1 to 8.5 grams/kg flour). The results of the eight events with highest expression levels are shown in the following table (Table 1).

**Table 1. Recombinant HSA expression level in top eight events**

| Line Number | Express Level (g/kg flour) |
|---|---|
| 508-3 | 5.5 |
| 508-17 | 8.0 |
| 508-71 | 8.7 |
| 508-73 | 4.0 |
| 508-77 | 8.5 |
| 508-83 | 8.5 |
| 508-101 | 3.0 |
| 508-113 | 4.0 |

### Example 4: Purification of recombinant human lactoferrin as cell culture media component

To prepare cell culture media supplemented with recombinant human lactoferrin, recombinant human lactoferrin was purified from rice flour. Transgenic rice line (164-12) expressing high levels of rhLF was selected. This line, now named as LF164, was planted two generations per year, alternating field planting In summer and greenhouse planting in winter. For protein purification, paddy rice expressing rhLF was de-hulled (Rice Mill, PS-160, Rimac, FL), and then ground to flour (average particle size of 100 mesh) using a hammer mill (8WA, Schutte-Buffalo, NY).

Protein extraction from transgenic flour was performed by mixing two kg of rice flour and 20 L of extraction buffer (0.02 M sodium phosphate pH 6.5 and 0.3 M sodium chloride) in a 50 L tank for 1 h. At the end of the mixing period, the suspension was allowed to settle overnight or centrifuged at 3750 rpm. In both cases, the supernatant was filtered through a plate and frame filter press (Ertel Alsop, 8S, NY) using M-05 and M-70 cellulose/perlite-based filters (Ertel Alsop, NY), respectively.

The filtrate containing rhLF and other rice flour soluble proteins was loaded onto an ion exchange column for further purification. An INDEX 200/500 process column (Amersham Pharmacia Biotech, NJ) packed with SP-Sepharose fast flow (Amersham Pharmacia Biotech, NJ) was used. The column was run at a linear flow rate of 150-200 cm/h. Packing, cleaning and testing of the resin bed was executed per manufacturer's instruction (HETP Test). The filtrate was loaded on to the resin at a linear velocity of 175 cm/h and washed with 0.02 M sodium phosphate buffer (pH 6.5) containing 0.3 M NaCl until the A₂₈₀ returned to baseline. Recombinant hLF was eluted using 20 mM sodium phosphate buffer (pH 6.5) containing 0.8 M NaCl. The washing and elution were performed at 200 cm/h and 150 cm/h, respectively.

A Centramate module (Pall Biopharmaceutical, MA) with 1 ft² 50 kDa polyethersulfone (Pall Biopharmaceutical, MA) membrane was used for concentration and desalting (ultrafiltration) of eluted hLF. The filtration was performed at a cross flow rate of approximately 1.5 L/min and an average transmembrane pressure of 10 psig. The eluted rhLF was concentrated and desalted to a final volume of 0.25 L and then lyophilized dry. Usually, about 3 grams of purified recombinant human lactoferrin was recovered from one kilogram of transgenic rice flour.

The recombinant human lactoferrin purified from rice flour is approximately 50% saturated with iron (partial-lactoferrin). The 50% saturated recombinant human lactoferrin was then made >90% iron saturated by iron up taking treatment, resulting in holo-lactoferrin and was made <10% iron saturated by acid treatment to remove bound iron resulting in apo-lactoferrin. The purified lactoferrins (holo-, partial- and apo-) were used as cell culture media components.

### Example 5: Cell culture media supplemented with recombinant human lactoferrin promotes cell growth,

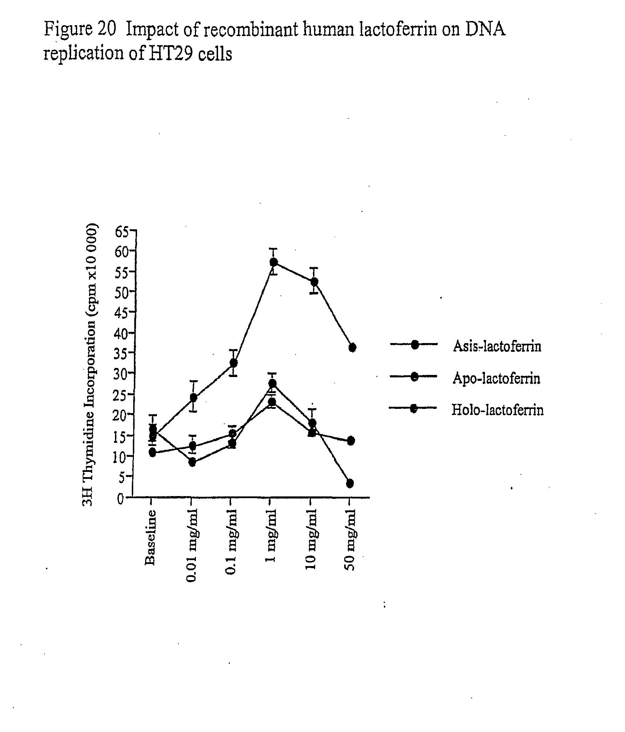

Recombinant human lactoferrin was added to minimal essential media (MEM) at various concentrations. This allows the test of growth factor effect of lactoferrin. The human colonic cancer cell line, HT-29, were maintained in MEM basic media containing 10% fetal calf serum (FCS). Lactoferrin samples were subsequently tested under serum-free conditions (MEM without supplementation of fetal calf serum). To assess the percentage of cells entering DNA synthesis, [³H]-thymidine (2 µCi/well) was included twenty four hours after the addition of lactoferrin samples and cells were left for a further 24 h. For each condition, the stimulatory or inhibitory effect of the lactoferrin solutions was measured in duplicate in six separate wells. Cell viability, determined by the ability to exclude 0.2% trypan blue, was greater than 90%. Proliferation assays reveal a typical 'bell shaped' dose response curve (Figure 20). Results demonstrated these responses to lactoferrin addition and show that maximal activity of lactoferrin was at a final concentration of 1 mg/ml, for all three forms of lactoferrin. Furthermore, of the three forms, the holo-lactoferrin showed the greatest proliferative activity (Figure 20).

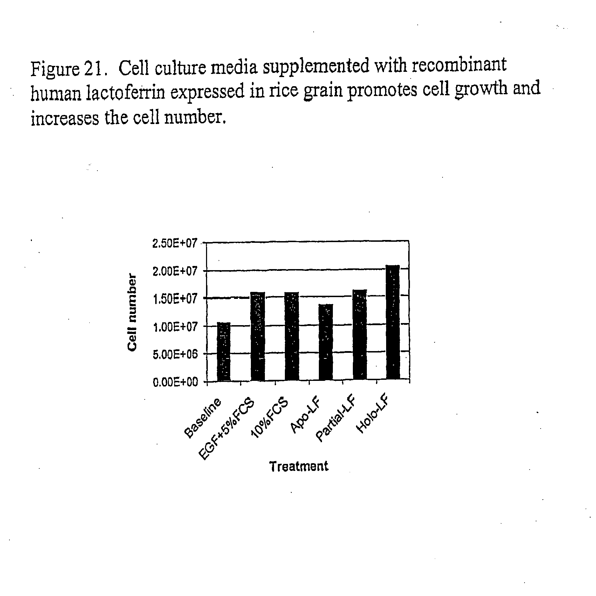

To determine cell viability, HT-29 Cells were seeded in 24-well plates at 5 x 10⁵ cells per well in MEM supplemented with 5% fetal calf serum (FCS); this resulted in an attached cell number of >95% per well 24h later (as assessed by trypsinization and direct counting using a Neubauer haemocytometer). At this time, the medium was changed in identically seeded wells to one containing MEM with 5 % FCS alone or MEM with 5% FCS plus EGF, or additional 5% FCS or various forms of Lactoferrin (i.e., apo-, partial- or holo-) at a concentration of 1 mg/ml for a further 48h. At the end of this period, cell numbers were assessed. To ensure that any cells present in the supernatant at this time were included in the total cell count, supernatants were collected, centrifuged at 13,000 rpm (12,000 g) for 5 min and added to the cells following trypsinization. Result shows that recombinant human lactoferrin derived from rice promote cell growth. There were twice as many cells in wells supplemented with holo-lactoferrin as that in wells without lactoferrin (Figure 21). Both thymidine incorporation and cell count experiments indicate that recombinant human lactoferrin from rice can be added as a component to cell culture media for fast cell growth (Figures 20 and 21).

### Example 6: Cell culture media supplemented with recombinant human lactoferrin promotes hybridoma cell growth

Recombinant human holo-lactoferrin was added to serum free media at various concentrations ranging from 5 to 250 mg/ml for hybridoma cell culture. Since the recombinant human lactoferrin is iron-saturated, holo-lactoferrin can provide iron for cell growth. The hybridoma cell line AE1 (ATCC) was maintained in DMEM basic media containing 5% fetal bovine serum (FBS). Lactoferrin was tested under serum-free conditions (AFM6, KC Bio, Kansas) without supplementation of fetal bovine serum. The cells were subcultured from 5% FBS to serum free media over multiple passages. At each subculture, the cells were analyzed for total cell count and viability. In order to demonstrate that recombinant human lactoferrin can replace transferrin derived from animal source, transferrin of equal concentration was added to AFM6 media as the control. The results demonstrated that the recombinant human holo-lactoferrin can be used to replace animal-derived transferrin in cell culture media for hybridoma cell growth.

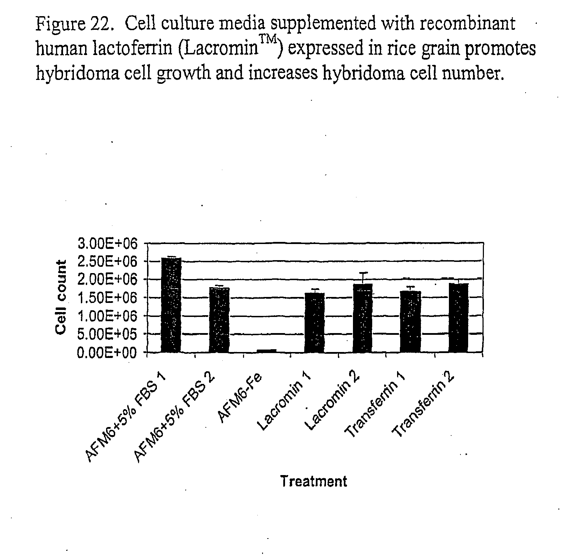

To determine the ability of holo-lactoferrin to promote cell growth, AE1 cells were sub-cultured from media containing 5% FBS to serum free media in T25 stationary flasks. The serum free media was supplemented with either animal derived transferrin; which is commonly used in the industry, or recombinant human holo-lactoferrin (LACROMIN™). This allows for a direct comparison between recombinant human holo-lactoferrin and animal derived transferrin. Each protein test was performed in triplicate with seeding densities of 5 X10⁵ cells per ml. To the subculture, the cells were collected by centrifugation and then seeded into fresh media with identical concentrations of either transferrin or recombinant human holo-lactoferrin (LACROMIN™). Each flask was analyzed on day 1 and day 3, following the subculture, for cell number and viability. An average cell count is given in Figure 22 for two passages. As can be seen, the cell viability and cell number when using recombinant human holo-lactoferrin are much higher than that without lactoferrin and equivalent to that of animal derived transferrin, indicating that plant-derived recombinant human holo-lactoferrin can promote hybridoma cell growth and replace animal derived transferrin.

The same analysis was carried out with another hybridoma cell line, L243. As can be seen in Table 2. LACROMIN^{™} (rhLF) can promote cell growth as as well as human transferrin (TF). L243 is also an IgG producing cell line. Samples were taken for IgG production. In medium containing LACROMIN^{™}, more IgG is produced than in medium containing transferrin.

**Table 2. Effect of human transferrin (TF) and recombinant human lactoferrin (rhLF) on hybridoma growth and monoclonal antibody production.**

| | TF/rhLF | Cell count (X10⁶) | | IgG level (ug/ml) | |
|---|---|---|---|---|---|
| Treatment | (ug/ml) | Mean | SD | Mean | SD |
| Control | 0 | 0.39 | 0.18 | 15.78 | 5.63 |
| TF | 5 | 0.70 | 0.25 | 34.10 | 8.44 |
| rhLF | 5 | 0.63 | 0.10 | 43.58 | 8.24 |
| TF | 25 | 0.78 | 0.24 | 31.93 | 7.10 |
| rhLF | 25 | 0.81 | 0.20 | 36.10 | 7.54 |
| TF | 100 | 0.86 | 0.33 | 28.58 | 5.82 |
| rhLF | 100 | 0.78 | 0.26 | 29.73 | 4.28 |
| 5% FBS | 0 | 0.98 | 0.12 | 23.88 | 5.50 |

### Example 7: Purification and characterization of recombinant human serum albumin

To purify rHSA from rice grain, rice grain expressing rHSA was dehusked, ground to flour and mixed for 30 min. with rHSA extraction buffer (Sodium Acetate, pH 4.9). After clarification by filtration, the filtrate was loaded onto an anion-exchange column (Q-Sepharose; GE Healthcare) at a rate of approximately 150 cm/hr. Once loaded, the resin was then washed with equilibration buffer to remove any unbound protein. The eluted rHSA is approximately 90% pure as analyzed by SDS-PAGE. Blue-Sepharose (GE Healthcare) can then be used to increase the purity from 90% to approximately 98% based on scanning analysis of SDS-PAGE gels. The rHSA is then concentrated and desalted via ultrafiltration prior to freeze drying.

Purified rHSA was subjected to a range of biochemical and biophysical characterizations for comparison to pHSA (Plasma-derived HSA) (Table 3). These tests indicate that rHSA expressed in rice is equivalent to pHSA.

**Table 3. Properties of rHSA as compared to pHSA**

| Property | pHSA | rHSA |
|---|---|---|
| N-terminal sequence | DAHKSE | DAHKSE |
| Glycosylation | None | None |
| SDS-PAGE/Western | ∼66 kDa | ∼66 kDa |
| Molecular mass (MALDI) | 66.9-68.1 kDa | 66.8-67.9 kDa |
| Isoelectric focusing point | pl 5.3 | pl 5.3 |
| Ligand binding | Yes | Yes |
| Thermal stability | Midpoint 65°C | Midpoint 65°C |
| Esterase activity | Yes | Yes |
| Protease sensitivity | Same as each other | Same as each other |

### Example 8: Cell culture media supplemented with recombinant human serum albumin promotes hybridoma cell growth in reduced -serum media.

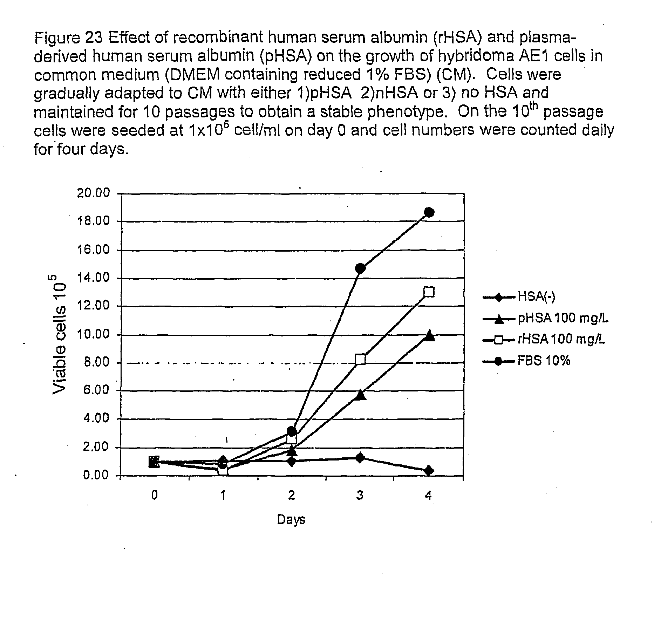

The effectiveness of recombinant human serum albumin from rice (rHSA) to stimulate cell growth similar to plasma derived human serum albumin (pHSA) was tested. Basal medium DMEM supplemented with 10 µg/ml insulin, 5.5 µg/ml transferrin, 6.7 ng/ml sodium selenite, 0.20 mg/ml ethanolamine and 2 mM Glutamax (Gibco) was used as common medium (CM). Hybridoma AE1 stocks in CM + 10% fetal bovine serum (FBS), were divided into four lineages: 1) CM +10% FBS as positive control 2) CM without HSA as negative control 3) CM + 100 mg/L rHSA as experimental, and 4) CM +100mg/L plasma-derived HSA (pHSA) as comparative control. Each lineage was then gradually adapted to 1% FBS (reduced serum) by sequential passage of 1x10⁵ cell/ml into medium containing 50% reduced FBS. At 1% FBS, each lineage was subsequently passaged 10 times to allow for full adaptation to growth under reduced-serum (1% FBS) conditions. Figure 23 shows cell growth curves of the 4 lineages during the 10th passage under CM +1%FBS. It was found that AE1 hybridoma cells grew much faster and reached a higher density in CM rHSA than in CM without any HSA (Figure 23). It was also found that cells grown in media with rHSA supplementation grew equivalently or better than cells grown in media with plasma derived HSA. This result indicates that rHSA from rice is functionally equivalent to pHSA in enhancing hybridoma cell growth in reduced-serum media.

### Example 9: Cell culture media supplemented with recombinant human serum albumin promotes hybridoma cell growth in serum-free media.

In order to demonstrate that plant derived recombinant human serum albumin functions equivalently to albumin derived from animals to support cell growth, the growth of AE1 hybridoma cells In supplemented serum-free media (SFM) was tested. SFM was prepared that was supplemented with either recombinant human serum albumin or plasma derived human serum albumin at similar concentrations (100 mg/L). SFM without supplemental albumin was prepared as a negative control. This approach allows for direct comparison between plant derived recombinant human serum albumin (CELLASTIM™) and plasma derived human serum albumin in promoting cell growth. SFM consisted of DMEM/F12 basal media supplemented with 10 µg/ml insulin, 5.5 µg/ml transferrin, 6.7 ng/ml sodium selenite, 0.20 mg/ml ethanolamine and 2 mM Glutamax (Gibco). To reach serum-free growth conditions, AE1 Hybridoma cells (ATCC # HB-72) were first adapted to SFM without FBS by a serial adaptation procedure. Briefly, AE1 grown in SFM/10% FBS supplemented with recombinant human serum albumin or plasma derived human serum albumin or no HSA, were successively subcultured at 1x10⁵ cells/ml into SFM with 50% reduced serum from the previous culture. The SFM/0.25% FBS cultures were subcultured directly into SFM without FBS. Six successive subcultures followed in SFM to allow cells to fully adapt to the respective SFM without FBS.

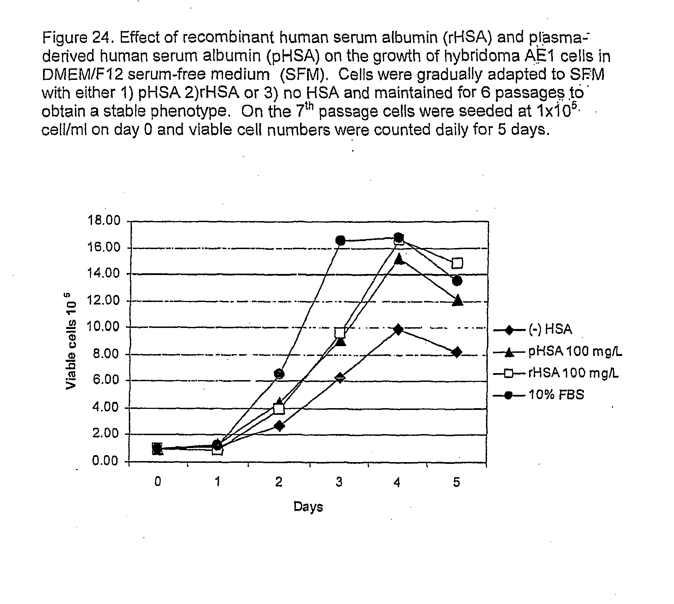

AE1 hybridoma cells grown for 6 passages in SFM containing 100 mg/L recombinant human serum albumin or 100 mg/L plasma derived human serum albumin or no HSA were sub-cultured 6-well plates at 1x10⁵ cells/ml. The number of viable cells and percent viability was determined daily to examine growth kinetics of the cultures. The subcultures were performed in duplicate to ensure an accurate viable cell count and analysis. Figure 24 shows that AE1 hybridoma cell grew faster and that the maximum number of viable cells was higher in medium supplemented with plant derived recombinant human serum albumin and equivalent to that of plasma derived human serum albumin. Thus, this example shows that plant derived recombinant human serum albumin can promote cell growth in serum-free media and replace animal derived albumin for use in tissue culture.

The invention now being fully described, it will be apparent to one of ordinary skill in the art that many changes and modifications can be made thereto without departing from the spirit or scope of the appended claims.

## Claims

1. Use of a cell culture media supplement comprising at least one plant-produced heterologous protein, as a component in a cell culture media, wherein the protein is recombinant transferrin produced in a plant, to improve growth and productivity of cells compared to use of media not containing the supplement.

2. The use of claim 1, wherein the recombinant transferrin is produced in a monocot plant selected from the group consisting of rice, barley, wheat, rye, corn, millet, triticale, or sorghum.

3. The use of claim 2, wherein the monocot plant is rice.

4. The use of claim 2, wherein the recombinant transferrin is produced in monocot seed and comprises about 3 % or greater of the total soluble protein in the monocot seed.

5. The use of claim 4, wherein the recombinant transferrin is expressed in the monocot using an expression vector comprising a promoter derived from a gene encoding a maturation specific monocot plant storage protein, a first DNA sequence, operably linked to the promoter, encoding a monocot plant seed-specific signal sequence capable of targeting a polypeptide linked thereto to an endosperm cell, and a second DNA sequence, linked in translation frame with the first DNA sequence, encoding transferrin.

6. The use of claim 5, wherein the promoter is derived from a gene encoding a maturation specific monocot plant storage protein selected from rice glutelins, globulins, oryzins and prolamines, barley hordeins, wheat gliadins and glutelins, maize zeins and glutelins, oat glutelins, sorghum kafirins, millet pennisetins, and rye secalins.

7. The use of claim 1, wherein the recombinant transferrin is produced in a dicot plant.

8. Use of the cell culture media supplement of any of claims 1 to 7, for cell culture where the cell culture media is a reduced serum or serum free media, or chemically defined media.

9. Use of the cell culture media supplement of any of claims 1 to 7, for cell culture where the cell culture media is a complete media, basal media, or basal media supplemented with the cell culture media component.

10. Use of the cell culture media supplement of any of claims 1 to 7, for cell culture where the cell culture media is derived exclusively from non-animal sources.
